(19) Europäisches Patentamt
European Patent Office
Office européen des brevets

(11) **EP 3 435 202 A1**

(12) **EUROPÄISCHE PATENTANMELDUNG**

(43) Veröffentlichungstag:
**30.01.2019 Patentblatt 2019/05**

(51) Int Cl.:
**G06F 3/01** (2006.01)  **A61B 34/00** (2016.01)
**G06K 9/00** (2006.01)  **G06F 3/0481** (2013.01)

(21) Anmeldenummer: **17183006.0**

(22) Anmeldetag: **25.07.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder:
• **Bagrecha, Nitin**
  **90402 Nuremberg (DE)**
• **Ebert, Anton**
  **90429 Nürnberg (DE)**
• **Laermann, Barbara**
  **80339 M?nchen (DE)**
• **von Wendorff, Cynthia**
  **91080 Spardorf (DE)**

Bemerkungen:
Geänderte Patentansprüche gemäss Regel 137(2) EPÜ.

(54) **ZUORDNEN EINES WERKZEUGES ZU EINER GREIFGESTE**

(57) Die Erfindung betrifft ein Verfahren zum Zuordnen eines ersten Werkzeuges zu einer Greifgeste, umfassend ein erstes Bestimmen einer ersten Position eines Daumens einer Hand einer Person und einer ersten Position eines Zeigefingers der Hand zu einem ersten Zeitpunkt mittels eines ersten Positionsdetektors, ein erstes Berechnen eines ersten Abstandes zwischen dem Daumen und dem Zeigefinger zu dem ersten Zeitpunkt basierend auf der ersten Position des Daumens und der ersten Position des Zeigefingers mittels einer Recheneinheit, ein zweites Bestimmen einer zweiten Position des Daumens und einer zweiten Position des Zeigefingers zu einem zweiten Zeitpunkt mittels des ersten Positionsdetektors, wobei der zweite Zeitpunkt zeitlich nach dem ersten Zeitpunkt liegt, ein zweites Berechnen eines zweiten Abstandes zwischen dem Daumen und dem Zeigefinger zu dem zweiten Zeitpunkt basierend auf der zweiten Position des Daumens und der zweiten Position des Zeigefingers mittels der Recheneinheit, ein erstes Identifizieren einer Greifgeste basierend auf dem ersten Abstand und dem zweiten Abstand mittels der Recheneinheit, ein Auswählen eines ersten Werkzeuges aus einer Werkzeugmenge basierend auf einer Werkzeugposition des ersten Werkzeuges mittels der Recheneinheit und ein Zuordnen des ersten Werkzeuges zu der Greifgeste mittels der Recheneinheit.

FIG 3

EP 3 435 202 A1

**Beschreibung**

In vielen Bereichen der Technik, insbesondere in der Medizintechnik, werden Schulungen und Trainings für Arbeitsabläufe vom Anwender nicht am echten Objekt (z.B. dem Patienten oder einer technischen Vorrichtung), sondern an Trainingsobjekten, in einer virtuellen Realität (ein englischer Fachbegriff ist "Virtual Reality", kury "VR"), in einer gemischten Realität (ein englischer Fachbegriff ist "Mixed Reality", kurz "MR") oder in einer erweiterten Realität (ein englischer Fachbegriff ist "Augmented Reality", kurz "AR") durchgeführt. Derartige Schulungen oder Trainings können durch Sensoren überwacht werden und automatisiert ausgewertet werden, hierdurch kann beispielsweise der Trainingserfolg automatisiert bewertet werden, oder das Training an die Erfordernisse und Leistungen des Anwenders angepasst werden.

Um eine automatisierte Auswertung zu ermöglichen, oder auch um eine Schulung oder ein Training in einer virtuellen, gemischten oder erweiterten Realität darzustellen, muss die korrekte Version bzw. ein Prototyp des Arbeitsablaufes aufgezeichnet werden. Hierfür wird regelmäßig ein bereits geschulter oder trainierter Anwender bei der Durchführung des Arbeitslaufes beobachtet bzw. seine Bewegungen aufgezeichnet. Bei der Übertragung des prototypischen Arbeitsablaufes in eine Schulung oder ein Training in der virtuellen Realität müssen in diesem Fall die beim prototypischen Arbeitsablauf durch den Anwender verwendeten Werkzeuge identifiziert werden.

Hierbei ist es bekannt, die Werkzeuge anhand einer optischen Aufnahme des Arbeitsablaufes (z.B. eines Filmes) manuell Zeitpunkten zuzuordnen, zu denen sie durch den geschulten oder trainierten Anwender aufgenommen wurden. Basierend auf diesen Zeitpunkten und den zugeordneten Werkzeugen kann dann ein Training oder eine Schulung erzeugt werden. Dieses manuelle Vorgehen ist aber zeitaufwändig und fehleranfällig, insbesondere bei kleinen Werkzeugen oder einer Vielzahl von zur Verfügung stehenden Werkzeugen muss die optische Aufnahme des Arbeitsablaufes auch eine sehr gute Qualität aufweisen, um die aufgenommenen Werkzeuge zu identifizieren.

Es ist daher die Aufgabe der Erfindung, bei der Aufzeichnung einer Schulung oder eines Trainings die während des prototypischen Arbeitsablaufes durch einen Anwender verwendeten Werkzeuge automatisiert, und damit insbesondere schneller und zuverlässiger zu identifizieren.

Die Aufgabe wird gelöst durch ein Verfahren zum Zuordnen eines ersten Werkzeuges nach Anspruch 1, durch ein Verfahren zum Bereitstellen einer Ablaufliste für eine Anwenderschulung, durch eine Zuordnungseinheit nach Anspruch 12, durch eine Bereitstellungseinheit, durch ein Computerprogrammprodukt nach Anspruch 14 und durch ein computerlesbares Speichermedium nach Anspruch 15. Vorteilhafte Weiterbildungen sind in der Beschreibung und den abhängigen Ansprüchen beschrieben.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Das erfindungsgemäße Verfahren zum Zuordnen eines ersten Werkzeuges zu einer Greifgeste basiert darauf, dass eine erste Position eines Daumens einer Hand einer Person und eine erste Position eines Zeigefingers der Hand zu einem ersten Zeitpunkt mittels eines ersten Positionsdetektors bestimmt werden. Weiterhin wird ein erster Abstand zwischen dem Daumen und dem Zeigefinger zu dem ersten Zeitpunkt basierend auf der ersten Position des Daumens und der ersten Position des Zeigefingers mittels einer Recheneinheit berechnet. Weiterhin wird eine zweite Position des Daumens und eine zweite Position des Zeigefingers zu einem zweiten Zeitpunkt mittels des ersten Positionsdetektors bestimmt, wobei der zweite Zeitpunkt zeitlich nach dem ersten Zeitpunkt liegt. Weiterhin wird ein zweiter Abstand zwischen dem Daumen und dem Zeigefinger zu dem zweiten Zeitpunkt basierend auf der zweiten Position des Daumens und der zweiten Position des Zeigefingers mittels der Recheneinheit bestimmt, wobei der zweite Zeitpunkt zeitlich nach dem ersten Zeitpunkt liegt. Weiterhin wird eine Greifgeste basierend auf dem ersten Abstand und dem zweiten Abstand mittels der Recheneinheit identifiziert. Weiterhin wird ein erstes Werkzeug aus einer Werkzeugmenge basierend auf einer Werkzeugposition des ersten Werkzeuges mittels der Recheneinheit ausgewählt. Weiterhin wird das erste Werkzeug mittels der Recheneinheit zu der Greifgeste zugeordnet.

Die Erfinder haben erkannt, dass durch die Erfassung der Position von Daumen und Zeigefinger und die Berechnung des Abstandes besonders einfach erkannt werden kann, dass eine Person einen Gegenstand ergreift. Wird eine solche Greifgeste oder ein solcher Vorgang des Ergreifens basierend auf dem Abstand registriert, kann das ergriffene Werkzeug besonders einfach und schnell aufgrund der Werkzeugposition aus einer Menge von Werkzeugen ausgewählt und damit der Greifposition zugeordnet werden.

Gemäß einem weiteren möglichen Aspekt der Erfindung entsprechen die erste Position und die zweite Position des Daumens den Koordinaten eines ausgezeichneten Punktes des Daumens, weiterhin vorteilhaft entsprechen die erste Position und die zweite Position des Zeigefingers den Koordinaten eines ausgezeichneten Punktes des Zeigefingers. Der erste Abstand zwischen Daumen und Zeigefinger zum ersten Zeitpunkt kann dann insbesondere dem euklidischen Abstand der ersten Position des Daumens von der ersten Position des Zeigefingers entsprechen, der zweite Abstand zwischen Daumen und Zeigefinger zum zweiten Zeitpunkt kann dann insbesondere dem euklidischen Abstand der zweiten Position des Daumens von der zweiten Position des Zeigefingers entsprechen.

Gemäß einem weiteren Aspekt der Erfindung wird die Greifgeste identifiziert, wenn der erste Abstand größer als ein Daumen-Zeigefinger-Abstandsschwellenwert (kurz "DZ-Abstandsschwellenwert") ist, und wenn der zweite Abstand kleiner oder gleich dem DZ-Abstandsschwellenwert ist. Die Erfinder haben erkannt, dass eine Greifgeste besonders einfach und zuverlässig identifiziert werden kann, wenn der Abstand von Daumen und Zeigefinger unter einen DZ-Abstandsschwellenwert fällt. Insbesondere kann der DZ-Abstandsschwellenwert zwischen 0,5 cm und 5 cm, insbesondere zwischen 0,5 cm und 3 cm, insbesondere zwischen 1cm und 2,5 cm, insbesondere 2 cm betragen. Die Erfinder haben erkannt, dass mit einem derartigen DZ-Abstandsschwellenwert eine besonders zuverlässige Identifizierung einer Greifgeste möglich ist.

Gemäß einem weiteren möglichen Aspekt der Erfindung ist der zeitliche Abstand zwischen dem ersten Zeitpunkt und dem zweiten Zeitpunkt kleiner als 2 Sekunden (kurz "2 s"), insbesondere kleiner als 1s, insbesondere kleiner als 100 Millisekunden (kurz "100 ms"), insbesondere kleiner als 10 ms. Insbesondere kann der zeitliche Abstand zwischen dem ersten Zeitpunkt und dem zweiten Zeitpunkt dem Inversen der Abtastfrequenz des ersten Positionsdetektors entsprechen. Die Erfinder haben erkannt, dass durch eine derartige Wahl des zeitlichen Abstands die Greifgeste besonders zuverlässig erkannt werden kann, da eine derartige Positionsveränderung zwischen Daumen und Zeigefinger in einem derartigen Zeitintervall sehr zuverlässig auf eine Greifbewegung oder Greifgeste der Person schließen lässt.

Gemäß einem weiteren möglichen Aspekt der Erfindung basiert das Auswählen weiterhin auf einer Handposition, wobei die Handposition die Position der Hand der Person zu einem dritten Zeitpunkt betrifft. Insbesondere kann die Handposition auf der ersten Position des Daumens und/oder auf der ersten Position des Zeigefingers basieren. Insbesondere kann die Handposition auch auf der zweiten Position des Daumens und/oder auf der zweiten Position des Zeigefingers basieren. Insbesondere kann die Handposition auch identisch mit der ersten Position des Daumens, der zweiten Position des Daumens, der ersten Position des Zeigefingers oder der zweiten Position des Zeigefingers sein. Der dritte Zeitpunkt kann insbesondere identisch mit dem ersten Zeitpunkt oder dem zweiten Zeitpunkt sein, weiterhin kann der dritte Zeitpunkt auch zwischen dem ersten und dem zweiten Zeitpunkt liegen. Der dritte Zeitpunkt kann auch als Greifgestenzeitpunkt bezeichnet werden. Die Erfinder haben erkannt, dass basierend auf der Handposition das erste Werkzeug besonders zuverlässig und mit wenigen Fehlern ausgewählt werden kann.

Gemäß einem weiteren möglichen Aspekt der Erfindung wird weiterhin ein zweites Werkzeug aus der Werkzeugmenge basierend auf einer Werkzeugposition des zweiten Werkzeuges mittels der Recheneinheit ausgewählt. Weiterhin wird das zweite Werkzeug mittels der Recheneinheit zu der Greifgeste zugeordnet. Die Erfinder haben erkannt, dass durch die Auswahl und die Zuordnung von mehreren Werkzeugen zu einer Greifgeste seltener Werkzeuge zugeordnet werden, welcher die Person gar nicht ergreift, insbesondere wenn Werkzeuge einen geringen Abstand aufweisen. Durch die Zuordnung von einem ersten und einem zweiten Werkzeug ist das Verfahren daher weniger fehleranfällig.

Nach einem weiteren Aspekt der Erfindung basiert das Auswählen weiterhin auf der ersten Position des Daumens und/oder der ersten Position des Zeigefingers, oder das Auswählen basiert auf der zweiten Position des Daumens und/oder der zweiten Position des Zeigefingers. Insbesondere kann das Auswählen auf der ersten Position des Daumens basieren, insbesondere kann das Auswählen auf der ersten Position des Zeigefingers basieren, insbesondere kann das Auswählen sowohl auf der ersten Position des Daumens als auch auf der ersten Position des Zeigefingers basieren. Insbesondere kann das Auswählen auch auf der zweiten Position des Daumens basieren, insbesondere kann das Auswählen auf der zweiten Position des Zeigefingers basieren, insbesondere kann das Auswählen sowohl auf der zweiten Position des Daumens als auch auf der zweiten Position des Zeigefingers basieren. Die Erfinder haben erkannt, dass durch die Einbeziehung der Position des Daumens und/oder des Zeigefingers zum ersten Zeitpunkt das erste Werkzeug besonders schnell und effizient ausgewählt werden kann.

Nach einem weiteren Aspekt der Erfindung wird das erste Werkzeug derart ausgewählt, dass der Abstand der Werkzeugposition von der zweiten Position des Daumens und/oder von der zweiten Position des Zeigefingers kleiner ist als ein Finger-Werkzeug-Abstandsschwellenwert (kurz "FW-Abstandsschwellenwert"). Die Erfinder haben erkannt, dass durch die Verwendung eines Schwellenwerts für den Abstand eine besonders einfache und schnelle Auswahl des ersten Werkzeugs durchgeführt werden kann.

Nach einem weiteren möglichen Aspekt der Erfindung wird das erste Werkzeug derart ausgewählt, dass der Abstand der Werkzeugposition von der ersten Position des Daumens und/oder von der ersten Position des Zeigefingers kleiner ist als ein Finger-Werkzeug-Abstandsschwellenwert (kurz "FW-Abstandsschwellenwert"). Die Erfinder haben erkannt, dass durch die Verwendung eines Schwellenwerts für den Abstand eine besonders einfache und schnelle Auswahl des ersten Werkzeugs durchgeführt werden kann.

Nach einem weiteren Aspekt der Erfindung wird genau ein erstes Werkzeug ausgewählt, wobei der Abstand des ersten Werkzeuges von der zweiten Position des Daumens oder von der zweiten Position des Zeigefingers kleiner oder gleich dem Abstand jedes Werkzeuges der Werkzeugmenge von der zweiten Position des Daumens oder der zweiten Position des Zeigefingers ist. Insbesondere kann der Abstand des ersten Werkzeuges von der zweiten Position des Daumens kleiner oder gleich dem Abstand jedes Werkzeuges der Werkzeugmenge von der zweiten Position des Daumens sein. Insbesondere kann der Abstand des ersten Werkzeuges von der zweiten Position des Zeigefingers kleiner oder gleich dem Abstand jedes Werkzeuges der Werkzeugmenge von der zweiten Position des Zeigefingers sein. Die Erfinder haben erkannt, dass durch eine derartige Auswahl des ersten Werkzeuges die Auswahl besonders schnell und einfach ist, da ein ergriffenes Werkzeug im Allgemeinen nahe an Daumen und Zeigefinger der greifenden Hand ist.

Nach einem weiteren möglichen Aspekt der Erfindung wird die Werkzeugposition basierend auf einer Werkzeugpositionsmarke mittels eines zweiten Positionsdetektors bestimmt, wobei die Werkzeugpositionsmarle am ersten Werkzeug angeordnet ist. Die Erfinder haben erkannt, dass mittels einer Werkzeugpositionsmarke eine Bestimmung der Werkzeugposition besonders einfach und genau durchgeführt werden kann. Der zweite Positionsdetektor kann insbesondere identisch mit dem ersten Positionsdetektor sein, der zweite Positionsdetektor kann aber auch insbesondere verschieden vom zweiten Positionsdetektor sein.

Nach einem weiteren möglichen Aspekt der Erfindung umfasst die Werkzeugpositionsmarke einen RFID-Transponder, und der zweite Positionsdetektor umfasst ein RFID-Lesegerät. Hierbei ist RFID ein englisches Akronym für "Radio frequency identification", eine deutsche Übersetzung wäre "Identifizierung mittels elektromagnetischer Wellen". Insbesondere kann das RFID-Lesegerät auch an der Hand der Person angeordnet sein. Die Erfinder haben erkannt, dass durch die Verwendung eines Systems umfassend einen RFID-Transponder und ein RFID-Lesegerät besonders genau und einfach Werkzeuge erkannt und ausgewählt werden können.

Nach einem weiteren Aspekt der Erfindung wird die Werkzeugposition mittels eines optischen bildgebenden Systems bestimmt. Hierbei basiert die Werkzeugposition auf wenigstens einem optischen Bild des optischen bildgebenden Systems. Insbesondere kann die Werkzeugposition auch auf zwei optischen Bildern des optischen bildgebenden Systems basieren, wobei die zwei optischen Bilder aus unterschiedlichen Richtungen aufgenommen wurden. Insbesondere kann die Werkzeugposition auch auf drei optischen Bildern des optischen bildgebenden Systems basieren, wobei die drei optischen Bilder aus drei paarweise verschiedenen Richtungen aufgenommen wurden. Die Erfinder haben erkannt, dass durch die Verwendung eines optischen bildgebenden Systems die Werkzeuge der Werkzeugmenge nicht modifiziert werden müssen, insbesondere also keine Werkzeugpositionsmarke angebracht werden muss. Dadurch kann das Verfahren kostengünstiger ausgeführt werden.

Nach einem weiteren Aspekt der Erfindung wird das erste Werkzeug aus einer Untermenge der Werkzeugmenge ausgewählt, wobei jedes Werkzeug der Untermenge einen Abstand zu der Person aufweist, der kleiner ist als ein Personen-Werkzeug-Abstandsschwellenwert (kurz PW-Abstandsschwellenwert). Der Abstand eines der Werkzeuge zu der Position ist insbesondere der Abstand des Werkzeuges zu einem ausgezeichneten Punkt der Person, beispielsweise zum Körpermittelpunkt der Person. Der PW-Abstandsschwellenwert kann insbesondere kleiner sein als 100 cm, insbesondere kleiner als 50 cm, insbesondere auch kleiner als 20 cm. Die Erfinder haben erkannt, dass durch eine derartige Einschränkung die Grundmenge, aus der das erste Werkzeug ausgewählt wird, reduziert werden kann. Dies führt zu einer Verringerung der notwendigen Rechenzeit und/oder zu einer Beschleunigung des Verfahrens.

Nach einem weiteren Aspekt der Erfindung umfasst der erste Positionsdetektor ein optisches bildgebendes System, wobei beim ersten Bestimmen ein erstes optisches Bild aufgenommen wird und die erste Position des Daumens und/oder die erste Position des Zeigefingers basierend auf dem ersten optischen Bild bestimmt wird, und wobei weiterhin beim zweiten Bestimmen ein zweites optisches Bild aufgenommen wird und die zweite Position des Daumens und/oder die zweite Position des Zeigefingers basierend auf dem zweiten optischen Bild bestimmt wird. Die Erfinder haben erkannt, dass durch die Verwendung eines optischen Bildgebenden Systems nur geringe Modifikationen an der Person durchgeführt werden müssen. Dies führt auf der einen Seite zu geringeren Kosten, auf der anderen Seite wird die Person nicht durch an sie angebrachte technische Vorrichtung bei der Aufnahme eines Trainings oder einer Schulung gestört.

Nach einem weiteren Aspekt der Erfindung ist an dem Daumen eine erste Positionsmarke angeordnet, weiterhin ist an dem Zeigefinger eine zweite Positionsmarke angeordnet. Die erste und die zweite Position des Daumens werden hierbei durch Lokalisierung der ersten Positionsmarke bestimmt, weiterhin werden die erste und die zweite Position des Zeigefingers durch Lokalisierung der zweiten Positionsmarke bestimmt. Insbesondere kann die erste Position des Daumens mit der Position der ersten Positionsmarke zum ersten Zeitpunkt und die zweite Position des Daumens mit der Position der ersten Positionsmarke zum zweiten Zeitpunkt identifiziert werden. Insbesondere kann auch weiterhin die erste Position des Zeigefingers mit der Position der zweiten Positionsmarke zum ersten Zeitpunkt und die zweite Position des Zeigefingers mit der Position der zweiten Positionsmarke zum zweiten Zeitpunkt identifiziert werden. Die Erfinder haben erkannt, dass durch die Verwendung von Positionsmarken die Positionen von Daumen und Zeigefinger besonders genau bestimmt werden können. Die Erfinder haben erkannt, dass hierdurch zum einen der Abstand zwischen Daumen und Zeigefinger besonders genau bestimmt werden kann, andererseits kann auch der Abstand von Daumen oder Zeigefinger zu einem Werkzeug besonders genau bestimmt werden.

Nach einem weiteren Aspekt der Erfindung umfasst der erste Positionsdetektor ein optisches bildgebendes System, wobei die erste Positionsmarke und die zweite Positionsmarke in einem optischen Bild lokalisiert werden. Bei dem optischen bildgebenden System zusammen mit der ersten und der zweiten Positionsmarke kann es sich insbesondere um ein "Motion Capturing System" handeln (eine deutsche Übersetzung ist "Bewegungserfassungssystem"). Die Erfinder haben erkannt, dass durch die Verwendung eines derartigen Systems die Positionen von Daumen und Zeigefinger gleichzeitig genau und kostengünstig bestimmt werden können, da für die Positionsmarken insbesondere keine aktiven Komponenten verbaut werden müssen.

Die Erfindung betrifft weiterhin ein Verfahren zum Bereitstellen einer Ablaufliste für eine Anwenderschulung, umfassend die folgenden Verfahrensschritte:

- Erfassen von Bewegungen der Person mittels eines ersten Positionsdetektors,
- Drittes Bestimmen einer Werkzeugmenge, mit denen eine Person bei der Anwenderschulung interagieren kann, mittels eines zweiten Positionsdetektors,
- Zweites Identifizieren einer Greifgeste basierend auf der Bewegung eines Daumens einer Hand der Person und eines Zeigefingers der Hand der Person mittels einer Recheneinheit,
- Auswählen eines ersten Werkzeuges aus der Werkzeugmenge basierend auf einer Werkzeugposition des ersten Werkzeuges mittels der Recheneinheit,
- Zuordnen des ersten Werkzeuges zu der Greifgeste mittels der Recheneinheit,
- Bereitstellen einer Ablaufliste umfassend wenigstens den Zeitpunkt der Greifgeste und das zugeordnete Werkzeug mittels einer Schnittstelle.

Die Erfinder haben erkannt, dass durch die bereitgestellte Ablaufliste eine Anwenderschulung besonders einfach und schnell erstellt werden kann, da die von der Person aufgenommenen Werkzeuge nicht mehr manuell erfasst werden müssen.

Nach einem weiteren Aspekt des Verfahrens zum Bereitstellen einer Ablaufliste wird die Greifgeste basierend auf einem ersten Abstand zwischen Daumen und Zeigefinger zu einem ersten Zeitpunkt und einem zweiten Abstand zwischen Daumen und Zeigefinger zu einem zweiten Zeitpunkt identifiziert, wobei der zweite Zeitpunkt zeitlich nach dem ersten Zeitpunkt liegt, und wobei der Zeitpunkt der Greifgeste zwischen dem ersten Zeitpunkt und dem zweiten Zeitpunkt liegt. Die Erfinder haben erkannt, dass ein erstes Identifizieren basierend auf Abständen von Daumen und Zeigefinger besonders einfach und genau durchführbar ist.

Nach einem weiteren möglichen Aspekt der Erfindung basiert das Auswählen des ersten Werkzeuges weiterhin auf der Handposition der Hand. Die Handposition kann hierbei insbesondere auf der Position des Daumens oder des Zeigefingers basieren, insbesondere auf den Positionen zum ersten Zeitpunkt und/oder zum zweiten Zeitpunkt. Die Erfinder haben erkannt, dass basierend auf der Handposition ein besonders einfaches und genaues Auswählen des ersten Werkzeuges erfolgen kann.

Nach einem weiteren Aspekt des Verfahrens zum Bereitstellen einer Ablaufliste umfasst der Verfahrensschritt des zweiten Identifizierens die folgenden Verfahrensschritte des Verfahrens zum Zuordnen eines ersten Werkzeuges:

- Erstes Bestimmen einer ersten Position eines Daumens einer Hand einer Person und einer ersten Position eines Zeigefingers der Hand zu einem ersten Zeitpunkt mittels des ersten Positionsdetektors,
- Erstes Berechnen eines ersten Abstandes zwischen dem Daumen und dem Zeigefinger zu dem ersten Zeitpunkt basierend auf der ersten Position des Daumens und der ersten Position des Zeigefingers mittels der Recheneinheit,
- Zweites Bestimmen einer zweiten Position des Daumens und einer zweiten Position des Zeigefingers zu einem zweiten Zeitpunkt mittels des ersten Positionsdetektors, wobei der zweite Zeitpunkt zeitlich nach dem ersten Zeitpunkt liegt,
- Zweites Berechnen eines zweiten Abstandes zwischen dem Daumen und dem Zeigefinger zu dem zweiten Zeitpunkt basierend auf der zweiten Position des Daumens und der zweiten Position des Zeigefingers mittels der Recheneinheit,
- Erstes Identifizieren einer Greifgeste basierend auf dem ersten Abstand und dem zweiten Abstand mittels der Recheneinheit.

Die Erfinder haben erkannt, dass durch diese Ausgestaltung des zweiten Identifizierens die Ablaufliste besonders einfach und zuverlässig erstellt werden kann.

Nach einem weiteren Aspekt des Verfahrens zum Bereitstellen einer Ablaufliste sind die Verfahrensschritte analog zum Verfahren zum Zuordnen eines ersten Werkzeuges weitergebildet. Insbesondere kann ein Schritt des Verfahrens zum Bereitstellen einer Ablaufliste, der einem Schritt des Verfahrens zum Zuordnen eines ersten Werkzeuges entspricht, durch Merkmale der Beschreibung des Verfahrens zum Zuordnen eines ersten Werkzeuges und durch Unteransprüche rückbezüglich auf das Verfahren zum Zuordnen eines ersten Werkzeuges weitergebildet sein. Entsprechende Vorteile sind analog zu übertragen.

Die Erfindung betrifft weiterhin ein Zuordnungssystem zum Zuordnen eines ersten Werkzeuges zu einer Greifgeste, umfassend folgende Einheiten:

- Erster Positionsdetektor, ausgebildet zum ersten Bestimmen einer ersten Position eines Daumens einer Hand einer Person und einer ersten Position eines Zeigefingers der Hand zu einem ersten Zeitpunkt, weiterhin ausgebildet zum zweiten Bestimmen einer zweiten Position des Daumens und einer zweiten Position des Zeigefingers zu einem zweiten Zeitpunkt, wobei der zweite Zeitpunkt zeitlich nach dem ersten Zeitpunkt liegt,
- Recheneinheit, ausgebildet zum ersten Berechnen eines ersten Abstandes zwischen dem Daumen und dem Zeigefinger zu dem ersten Zeitpunkt basierend auf der ersten Position des Daumens und der ersten Position des Zeigefingers, weiterhin ausgebildet zum zweiten Berechnen eines zweiten Abstandes zwischen dem Daumen und dem Zeigefinger zu dem zweiten Zeitpunkt basierend auf der zweiten Position des Daumens und der zweiten Position des Zeigefingers,

weiterhin ausgebildet zum ersten Identifizieren einer Greifgeste basierend auf dem ersten Abstand und dem zweiten Abstand mittels der Recheneinheit,
weiterhin ausgebildet zum Auswählen eines ersten Werkzeuges aus einer Werkzeugmenge basierend auf einer Werkzeugposition des ersten Werkzeuges,
weiterhin ausgebildet zum Zuordnen des ersten Werkzeuges zu der Greifgeste.

Ein solches Zuordnungssystem kann insbesondere dazu ausgebildet sein, die zuvor beschriebenen erfindungsgemäßen Verfahren und ihre Aspekte auszuführen. Das Zuordnungssystem ist dazu ausgebildet diese Verfahren und ihre Aspekte auszuführen, indem der erste Positionsdetektor und die Recheneinheit ausgebildet sind, die entsprechenden Verfahrensschritte auszuführen.

Die Erfindung kann weiterhin ein Bereitstellungssystem betreffen, umfassend die folgenden Einheiten:

- Erster Positionsdetektor, ausgebildet zum Erfassen von Bewegungen einer Person,
- Zweiter Positionsdetektor, ausgebildet zum Dritten Bestimmen einer Menge von Werkzeugen, mit denen eine Person bei der Anwenderschulung interagieren kann,
- Recheneinheit, ausgebildet zum zweiten Identifizieren einer Greifgeste basierend auf der Bewegung eines Daumens einer Hand der Person und eines Zeigefingers der Hand der Person, weiterhin ausgebildet zum Auswählen eines ersten Werkzeuges aus einer Werkzeugmenge basierend auf einer Werkzeugposition des ersten Werkzeuges, weiterhin ausgebildet zum Zuordnen des ersten Werkzeuges zu der Greifgeste,
- Schnittstelle zum Bereitstellen einer Ablaufliste umfassend wenigstens den Zeitpunkt der Greifgeste und das zugeordnete Werkzeug.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Zuordnungssysteme auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Bei der Handposition handelt es sich insbesondere um die Position eines ausgezeichneten Punktes der Hand. Die Handposition kann insbesondere auf der ersten Position des Daumens und/oder des Zeigefingers oder alternativ auf der zweiten Position des Daumens und/oder des Zeigefingers basieren.

Bei der Werkzeugposition eines ersten Werkzeuges und/oder eines zweiten Werkzeuges handelt es sich insbesondere um die Position eines ausgezeichneten Punktes des ersten Werkzeuges und/oder des zweiten Werkzeuges.

Die erste Position (des Daumens und/oder des Zeigefingers), die zweite Position (des Daumens und/oder des Zeigefingers), die Handposition und/oder die Werkzeugposition können hierbei durch dreidimensionale Koordinaten des ausgezeichneten Punktes bezüglich eines Referenzkoordinatensystems charakterisiert sein. Weiterhin können die erste Position und/oder die zweite Position des Daumens auch eine Ausrichtung des Daumens umfassen, weiterhin können die erste Position und/oder die zweite Position des Zeigefingers auch eine Ausrichtung des Zeigefingers umfassen, weiterhin kann die Handposition auch eine Ausrichtung der Hand umfassen, weiterhin kann die Werkzeugposition auch eine Ausrichtung des Werkzeuges umfassen. Eine solche Ausrichtung kann durch dreidimensionale Koordinaten (eines Vektors) bezüglich des Referenzkoordinatensystems definiert sein. Die Werkzeugposition kann insbesondere auch durch einen ausgezeichneten Punkt einer Werkzeugpositionsmarke gegeben sein, wobei die Werkzeugpositionsmarke an dem Werkzeug angeordnet ist. Bei dem ausgezeichneten Punkt der Werkzeugpositionsmarke kann es sich insbesondere um den Mittelpunkt der Werkzeugpositionsmarke handeln.

Die erste Position (des Daumens und/oder des Zeigefingers), die zweite Position (des Daumens und/oder des Zeigefingers), die Handposition und/oder die Werkzeugposition können auch relativ zu einem anderen Punkt im Referenzkoordinatensystem definiert sein. Insbesondere können die erste Position (des Daumens und/oder des Zeigefingers), die zweite Position (des Daumens und/oder des Zeigefingers), die Handposition und/oder die Werkzeugposition auch nur eine Angabe über den jeweiligen Abstand zu dem anderen Punkt betreffen.

Bei einer ersten Positionsmarke, einer zweiten Positionsmarke und/oder einer Werkzeugpositionsmarke handelt es sich insbesondere um eine aktive Positionsmarke oder eine passive Positionsmarke. Eine aktive Positionsmarke kann insbesondere Strahlung aussenden, die eine Positionsbestimmung der aktiven Positionsmarke ermöglicht. Strahlung kann insbesondere Strahlung aus dem elektromagnetischen betreffen, hierbei kann Strahlung insbesondere Licht oder Radiostrahlung bezeichnen. Beispiele für aktive Positionsmarke sind RFID-Transponder oder Bluetooth-Beacons, ein Beispiel für eine passive Positionsmarke sind farbige Kugeln.

Bei einem optischen bildgebenden System kann es sich insbesondere um eine Kamera oder eine Mehrzahl von Kameras handeln, die mittels Licht im sichtbaren Spektrum Bilddaten generiert. Bei den Bilddaten kann es sich insbesondere um zweidimensionale und/oder dreidimensionale Bilddaten handeln. Insbesondere kann bei einer Mehrzahl von Kameras, die ein Objekt aus mehreren Richtungen aufnehmen, Bildpunkten der Bilddaten eine Koordinate im dreidimensionalen Raum zugeordnet werden.

Als Greifgeste wird hier insbesondere das Zusammenführen und/oder das Schließen von Daumen und Zeigefinger einer Hand einer Person bezeichnet, unabhängig davon, ob tatsächlich ein Werkzeug oder ein anderer Gegenstand aufgenommen wird. Einer Greifgeste kann ein Greifgestenzeitpunkt und eine Greifgestenposition zugeordnet werden.

Im Folgenden wird die Erfindung anhand von Figuren und Pseudocode näher erläutert und beschrieben. Es zeigen

Fig. 1     eine Flussdiagramm eines Verfahrens zur Zuordnung einer Greifgeste zu einem ersten Werkzeug,

Fig. 2     ein Zuordnungssystem,

Fig. 3     eine Hand einer Person zu einem ersten Zeitpunkt,

Fig. 4     die Hand der Person zu einem zweiten Zeitpunkt,

Fig. 5     eine Hand einer Person mit Positionsmarken,

Fig. 6     einen Werkzeugtisch mit Werkzeugen,

Fig. 7     eine schematische Darstellung des Aufbaus zur Durchführung des Verfahrens aus der Vogelperspektive,

Fig. 8     ein erstes Ausführungsbeispiel eines Bereichs von Werkzeugpositionen, die einer Greifgeste zugeordnet werden können,

Fig. 9     ein zweites Ausführungsbeispiel eines Bereichs von Werkzeugpositionen, die einer Greifgeste zugeordnet werden können.

Fig. 10     eine Zuordnungstabelle von Greifgesten zu Werkzeugen,

Fig. 11     ein Flussdiagramm eines Verfahrens zum Bereitstellen einer Ablaufliste für eine Anwenderschulung.

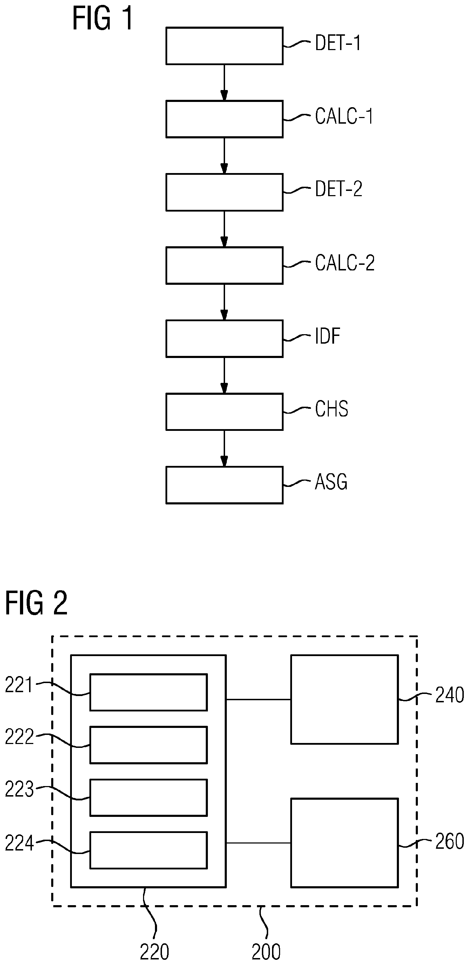

Fig. 1 zeigt ein Flussdiagramm eines Verfahrens zur Zuordnung einer Greifgeste zu einem ersten Werkzeug 601, 602, 603. Der erste Schritt des in der Fig. 1 dargestellten Verfahrens ist das erste Bestimmen DET-1 einer ersten Position 311.1 eines Daumens 301 einer Hand 300 einer Person 701 und einer ersten Position 312.1 eines Zeigefingers 302 der Hand 300 zu einem ersten Zeitpunkt mittels eines ersten Positionsdetektors 240. Im dargestellten Ausführungsbeispiel sind eine erste Positionsmarke 321 am Daumen 301 und eine zweite Positionsmarke 322 am Zeigefinger 302 angeordnet. Bei der ersten Positionsmarke 321 und der zweiten Positionsmarke 322 handelt es sich jeweils um ein kugelförmiges Objekt mit einer Farbe, die sich von der Farbe der Hand 300 unterscheidet, beispielsweise mit der Farbe weis.

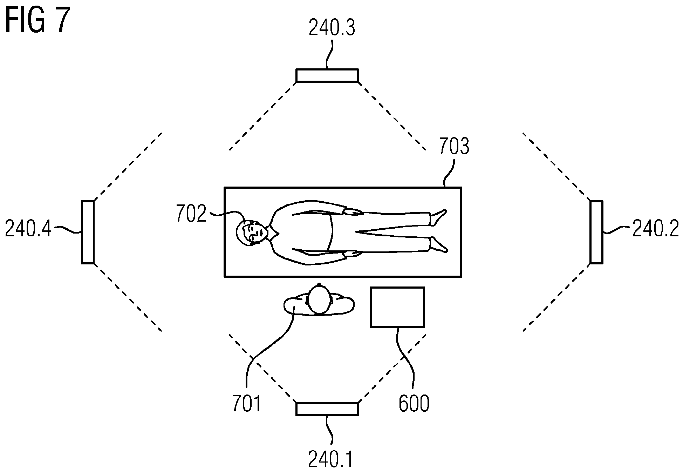

Beim ersten Positionsdetektor 240 handelt es sich im dargestellten Ausführungsbeispiel um ein System aus mehreren Teilpositionsdetektoren 240.1, ..., 240.4, wobei jeder der Teilpositionsdetektoren 240.1, ..., 240.4 ein optisches bildgebendes System ist, beispielsweise eine Kamera, welches dazu ausgebildet ist, Bilder im optischen Spektrum aufzunehmen. Die Teilpositionsdetektoren 240.1, ..., 240.4 sind derart angeordnet, dass die Hand 300 und damit die Positionsmarken 321, 322 aus mehreren Richtungen abgebildet werden können, und daher aus den zweidimensionalen Bilddaten der Teilpositionsdetektoren 240.1, ..., 240.4 die dreidimensionalen Koordinaten der Positionsmarken 321, 322 bestimmt werden können. Eine beispielhafte Anordnung der Teilpositionsdetektoren 240.1, ..., 240.4 ist der Fig. 7 zu entnehmen. Alternativ kann auch auf separate Positionsmarken 321, 322 verzichtet werden, in diesem Fall werden charakteristische Strukturen (z.B. der Fingernagel oder die Fingerspitze) des Daumens 301 oder des Zeigefingers 302 durch Bilderkennung in den Bildern lokalisiert und die dreidimensionale Position dieser charakteristischen Strukturen bestimmt.

Im dargestellten Ausführungsbeispiel wird die erste Position 311.1 des Daumens 301 mit dem Mittelpunkt der ersten Positionsmarke 321 zum ersten Zeitpunkt identifiziert. Weiterhin wird die erste Position 312.1 des Zeigefingers 302 mit dem Mittelpunkt der ersten Positionsmarke 322 zum ersten Zeitpunkt identifiziert. Demnach entspricht die erste Position 311.1 des Daumens 301 und die erste Position 312.1 des Zeigefingers 302 einem Koordinatentupel, insbesondere einem dreidimensionalen Koordinatentupel.

Der zweite Schritt des in der Fig. 1 dargestellten Verfahrens ist das erste Berechnen CALC-1 eines ersten Abstandes 315.1 zwischen dem Daumen 301 und dem Zeigefinger 302 zu dem ersten Zeitpunkt basierend auf der ersten Position 311.1 des Daumens 301 und der ersten Position 312.1 des Zeigefingers 302 mittels einer Recheneinheit 222. Im dargestellten Ausführungsbeispiel wird der erste Abstand 315.1 als euklidischer Abstand der ersten Position 311.1 des Daumens 301 zur ersten Position 312.1 des Zeigefingers 302 berechnet, in anderen Worten als

$$\mathrm{dist}(\mathrm{x},\mathrm{y}) := \sqrt{\left(x_1 - y_1\right)^2 + \left(x_2 - y_2\right)^2 + \left(x_3 - y_3\right)^2}$$

wobei x die erste Position 311.1 des Daumens 301 und y die erste Position 312.1 des Zeigefingers 302 bezeichnet, und wobei $x_i$ bzw. $y_i$ den i-ten Eintrag des jeweiligen Koordinatentupels bezeichnen.

Der dritte Schritt des in der Fig. 1 dargestellten Verfahrens ist das zweite Bestimmen DET-2 einer zweiten Position 311.2 des Daumens 301 und einer zweiten Position 312.2 des Zeigefingers 302 zu einem zweiten Zeitpunkt mittels des ersten Positionsdetektors 240, wobei der zweite Zeitpunkt zeitlich nach dem ersten Zeitpunkt liegt. Das zweite Bestimmen DET-2 ist vorteilhafterweise analog zum ersten Bestimmen DET-1 des dargestellten Ausführungsbeispiels ausgebildet. Der zeitliche Abstand der ersten Zeitpunktes und des zweiten Zeitpunktes kann beispielsweise als 0,1 Sekunden gewählt werden, alternativ sind natürlich auch andere Zeitabstände möglich.

Insbesondere wird im dargestellten Ausführungsbeispiel die zweite Position 311.2 des Daumens 301 mit dem Mittelpunkt der ersten Positionsmarke 321 zum zweiten Zeitpunkt identifiziert. Weiterhin wird die zweite Position 312.2 des Zeigefingers 302 mit dem Mittelpunkt der ersten Positionsmarke 322 zum zweiten Zeitpunkt identifiziert. Demnach entsprechen die zweite Position 311.2 des Daumens 301 und die zweite Position 312.2 des Zeigefingers 302 einem Koordinatentupel, insbesondere einem dreidimensionalen Koordinatentupel.

Es ist in einem alternativen Ausführungsbeispiel des Verfahrens ebenfalls möglich, dass das erste Berechnen CALC-1 zeitlich nach dem zweiten Bestimmen DET-2 durchgeführt wird, genauso ist es möglich, dass das erste Berechnen CALC-2 zeitgleich mit dem zweiten Bestimmen DET-2 durchgeführt wird. Der Schritt des zweiten Bestimmens DET-2 ist unabhängig vom Schritt des ersten Berechnens CALC-1.

Der vierte Schritt des in Fig. 1 dargestellten Verfahrens ist das zweite Berechnen CALC-2 eines zweiten Abstandes 315.2 zwischen dem Daumen 301 und dem Zeigefinger 302 zu dem zweiten Zeitpunkt basierend auf der zweiten Position 311.2 des Daumens 301 und der zweiten Position 312.2 des Zeigefingers 302 mittels der Recheneinheit 222. Im dargestellten Ausführungsbeispiel wird der zweite Abstand 315.2 als euklidischer Abstand der zweiten Position 311.2 des Daumens 301 zur zweiten Position 312.2 des Zeigefingers 302 berechnet.

Der fünfte Schritt des in Fig. 1 dargestellten Ausführungsbeispiels ist das erste Identifizieren IDF-1 einer Greifgeste basierend auf dem ersten Abstand 315.1 und dem zweiten Abstand 315.2 mittels der Recheneinheit 222. Im dargestellten Ausführungsbeispiel wird eine Greifgeste identifiziert, wenn der erste Abstand 315.1 größer ist als ein DZ-Abstandsschwellenwert, und wenn der zweite Abstand 315.2 kleiner oder gleich dem DZ-Abstandsschwellenwert ist. In anderen Worten wird eine Greifgeste erkannt, wenn der Abstand zwischen Daumen 301 und Zeigefinger 302 den DZ-Abstandsschwellenwert unterschreitet. Ein typischer und geeigneter DZ-Abstandsschwellenwert ist 2cm, es können aber natürlich auch andere DZ-Abstandsschwellenwerte verwendet werden. Der DZ-Abstandsschwellenwert kann vorteilhafterweise auch an die Größe der Hand 300 der Person 701 angepasst werden.

Der sechste Schritt des in Fig. 1 dargestellten Ausführungsbeispiels des Verfahrens ist das Auswählen CHS eines ersten Werkzeuges 601, 602, 603 aus einer Werkzeugmenge basierend auf einer Werkzeugposition des ersten Werkzeuges 601, 602, 603 mittels der Recheneinheit 222. Im dargestellten Ausführungsbeispiel ist die Werkzeugposition eines Werkzeuges 601, 602, 603 die Position des Werkzeuges 601, 602, 603 zum zweiten Zeitpunkt. Alternativ kann als Werkzeugposition eines Werkzeuges 601, 602, 603 auch die Position des Werkzeuges 601, 602, 603 zum ersten Zeitpunkt gewählt werden. Weiterhin alternativ kann als Werkzeugposition eines Werkzeuges 601, 602, 603 auch die Position des Werkzeuges 601, 602, 603 zu einem anderen Zeitpunkt zwischen dem ersten und dem zweiten Zeitpunkt gewählt werden, insbesondere zum zeitlichen Mittelpunkt des ersten Zeitpunktes und des zweiten Zeitpunktes.

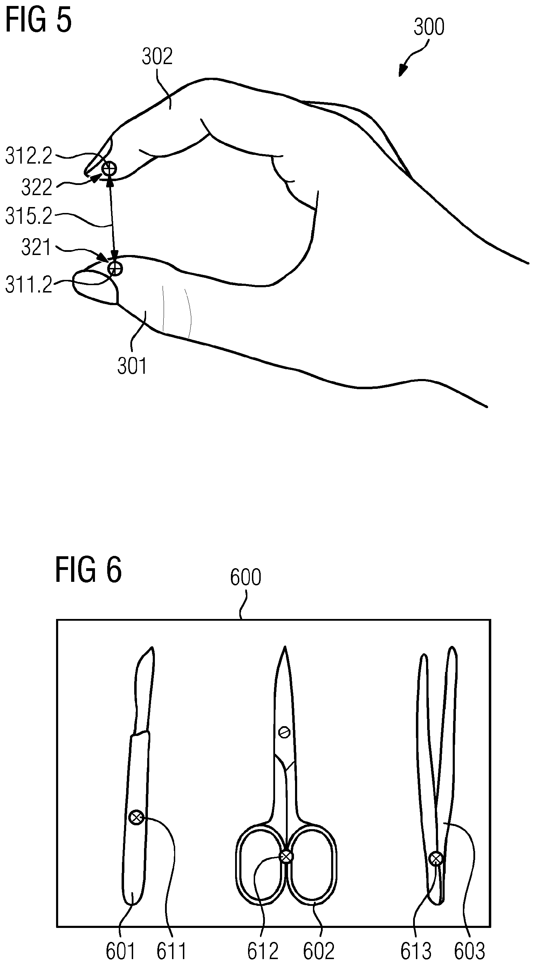

An jedem der Werkzeuge 601, 602, 603 ist in diesem Ausführungsbeispiel jeweils ein RFID-Transponder als Werkzeugpositionsmarke 611, 612, 613 angeordnet, dessen Position mit einem RFID-Lesegerät als zweitem Positionsdetektor 260 bestimmt werden kann. Für eine konkrete Beschreibung wird auf die Fig. 6 verwiesen.

Im dargestellten Ausführungsbeispiel wird dasjenige Werkzeug 601, 602, 603 aus der Werkzeugmenge ausgewählt, dessen Werkzeugposition welches einen minimalen Abstand zur zweiten Position 311.2 des Daumens 301 aufweist.

Der siebte Schritt des in Fig. 1 dargestellten Ausführungsbeispiels des Verfahrens ist das Zuordnen ASG des ersten Werkzeuges 601, 602, 603 zu der Greifgeste mittels der Recheneinheit 222. Im dargestellten Ausführungsbeispiel wird hierfür eine Zuordnung zwischen dem Zeitpunkt der Greifgeste und einem Werkzeugdatensatz 1021.1, ..., 1021.M in einer Ablaufliste und/oder einer Datenbank gespeichert. Optional können in dieser Ablaufliste und/oder dieser Datenbank noch weitere Daten gespeichert werden, z.B. die Handposition und/oder die Werkzeugposition zum Zeitpunkt der Greifgeste.

Diese Zuordnungen von einem oder mehreren ersten Werkzeugen 601, 602, 603 zu Greifgesten kann dann verwendet werden, um einen Sollablauf für eine Anwenderschulung bereitzustellen.

Fig. 2 zeigt ein Zuordnungssystem 200, umfassend einen ersten Positionsdetektor 240 sowie eine Zuordnungseinheit 220. Das hier dargestellte Zuordnungssystem 200 ist dazu ausgebildet, das erfindungsgemäße Verfahren auszuführen. Optional kann das Zuordnungssystem 200 auch einen zweiten Positionsdetektor 260 umfassen. Die Zuordnungseinheit 220 und der erste Positionsdetektor 240 bzw. der optionale zweite Positionsdetektor 260 sind zum Datenaustausch miteinander verbunden, bei einer derartigen Verbindung kann es sich um eine Netzwerkverbindung (z.B. ein Lokalnetzwerk über Ethernet, ein englischer Fachbegriff ist "Local Area Network", kurz "LAN"; ein drahtloses Funknetzwerk, ein englischer Fachbegriff ist "wireless LAN", kurz "WLAN") eine Verbindung über "universal serial Bus" (kurz "USB") oder um andere bekannte Verbindungen zum Anschluss von Peripheriegeräten an Computer handeln.

Die Zuordnungseinheit 220 umfasst eine Schnittstelle 221, eine Recheneinheit 222, eine Speichereinheit 223 sowie eine Ein- und Ausgabeeinheit 224. Bei der Zuordnungseinheit 220 kann es sich insbesondere um einen Computer, einen Mikrocontroller oder um einen integrierten Schaltkreis handeln. Alternativ kann es sich bei der Zuordnungseinheit 220 um einen realen oder virtuellen Verbund von Computern handeln (ein englischer Fachbegriff für einen realen Verbund ist "Cluster", ein englischer Fachbegriff für einen virtuellen Verbund ist "Cloud"). Bei einer Schnittstelle 221 kann es sich um eine Hardware- oder Softwareschnittstelle handeln (beispielsweise PCI-Bus, USB oder Firewire). Eine Recheneinheit 222 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit 223 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Eine Ein- und Ausgabeeinheit 224 umfasst wenigstens eine Eingabeeinheit und/oder wenigstens eine Ausgabeeinheit.

Im dargestellten Ausführungsbeispiel ist der erste Positionsdetektor 240 ein System aus mehreren Teilpositionsdetektoren 240.1, ..., 240.4, wobei jeder der Teilpositionsdetektoren 240.1, ..., 240.4 ein optisches bildgebendes System ist, beispielsweise eine Kamera, welches dazu ausgebildet ist, Bilder im optischen Spektrum aufzunehmen. Dieser erste Positionsdetektor 240 wurde bereits vorstehend näher beschrieben.

Weiterhin ist im dargestellten Ausführungsbeispiel der zweite Positionsdetektor 260 ein RFID-Lesegerät, der Signale von RFID-Transpondern empfangen und verarbeiten kann. Zur Bestimmung der Werkzeugposition ist an den zur Verfügung stehenden Werkzeugen 601, 602, 603 eine Werkzeugpositionsmarke 611, 612, 613 als RFID-Transponder ausgebildet.

Alternativ kann natürlich auch der zweite Positionsdetektor 260 analog zum ersten Positionsdetektor 240 dieses Ausführungsbeispiels durch mehrere optische bildgebende Systeme gebildet sein, ebenso kann alternativ der erste Positionsdetektor 240 als RFID-Lesegerät ausgebildet sein.

Wiederum alternativ sind auch für den ersten Positionsdetektor 240 und/oder den zweiten Positionsdetektor 260 auch andere Ausführungsformen vorstellbar, beispielsweise basierend auf Bluetooth-Beacons als Werkzeugpositionsmarken 611, 612, 613, vorteilhafterweise unter der Verwendung der Technologie "Bluetooth Low Energy", oder basierend auf anderen Sende- und Empfangsvorrichtungen für elektromagnetische Wellen, insbesondere Radiowellen.

Der zweite Positionsdetektor 260 kann auch identisch mit dem ersten Positionsdetektor 240 sein, wenn die Bestimmung der Positionen von Daumen 301 und Zeigefinger 302 analog zur Bestimmung einer Werkzeugposition durchgeführt wird.

Wenn der optionale zweite Positionsdetektor 260 nicht vorhanden ist, kann die Werkzeugposition von jedem der Werkzeuge 601, 602, 603 vorab festgelegt sein und in der Speichereinheit 223 hinterlegt werden. Dies ist beispielsweise vorteilhaft, wenn die Werkzeuge 601, 602, 603 vor der Aufzeichnung der Anwenderschulung an vorgegebenen Positionen im Raum angeordnet sind, und Werkzeuge 601, 602, 603 nach einer Verwendung durch die Person 701 an den Ausgangsort zurückgelegt werden. Dies ist beispielsweise bei medizinischen Anwendungen der Fall, bei denen die chirurgischen Instrumente in einer vorgegebenen Anordnung auf dem Werkzeugtisch 600 platziert sind.

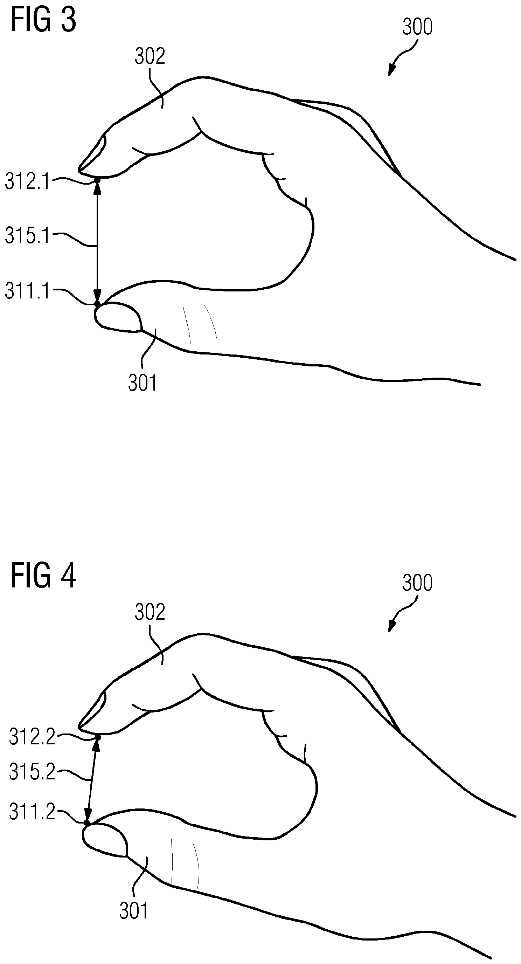

Fig. 3 zeigt eine Darstellung einer Hand 300 einer Person 701 zu einem ersten Zeitpunkt, Fig. 4 zeigt eine

EP 3 435 202 A1

Darstellung der Hand 300 der Person 701 zu einem zweiten Zeitpunkt. Die Hand 300 umfasst einen Daumen 301 und einen Zeigefinger 302. In Fig. 3 sind weiterhin die erste Position 311.1 des Daumens 301 und die erste Position 312.1 des Zeigefingers 302 dargestellt, wobei die erste Position 311.1 des Daumens 301 die Position des Daumens 301 zum ersten Zeitpunkt ist, und wobei die erste Position 312.1 des Zeigefingers 302 die Position des Zeigefingers 302 zum ersten Zeitpunkt ist, weiterhin ist der erste Abstand 315.1 zwischen der ersten Position 311.1 des Daumens 301 und der ersten Position 312.1 des Zeigefingers 302 dargestellt. In Fig. 4 sind weiterhin die zweite Position 311.2 des Daumens 301 und die zweite Position 312.2 des Zeigefingers 302 dargestellt, wobei die zweite Position 311.2 des Daumens 301 die Position des Daumens 301 zum zweiten Zeitpunkt ist, und wobei die zweite Position 312.2 des Zeigefingers 302 die Position des Zeigefingers 302 zum zweiten Zeitpunkt ist, weiterhin ist der zweite Abstand 315.2 zwischen der zweiten Position 311.2 des Daumens 301 und der zweiten Position 312.2 des Zeigefingers 302 dargestellt.

Im dargestellten Ausführungsbeispiel der Fig. 3 und der Fig. 4 ist die erste Position 311.1 des Daumens 301 durch einen ausgezeichneten Punkt des Daumens 301 gegeben, hier die Kante des Daumennagels. Die zweite Position 311.2 des Daumens 301 ist durch denselben ausgezeichneten Punkt gegeben. Weiterhin ist die erste Position 312.1 des Zeigefingers 302 ebenfalls durch einen ausgezeichneten Punkt gegeben, hier den Mittelpunkt der Fingerkuppe. Die zweite Position 312.2 des Zeigefingers 302 ist durch denselben ausgezeichneten Punkt gegeben. Der erste Abstand 315.1 entspricht hier dem euklidischen Abstand zwischen der ersten Position 311.1 des Daumens 301 und der ersten Position 312.1 des Zeigfingers 302. Der zweite Abstand 315.2 entspricht hier dem euklidischen Abstand zwischen der zweiten Position 311.2 des Daumens 301 und der zweiten Position 312.2 des Zeigefingers 302.

Fig. 5 zeigt die Hand 300 einer Person 701 in einem Ausführungsbeispiel mit einer ersten Positionsmarkierung 321 und einer zweiten Positionsmarkierung 322. Hierbei ist die erste Positionsmarkierung 321 am Daumen 301 angeordnet, weiterhin ist die zweite Positionsmarkierung 322 am Zeigefinger 302 angeordnet. In diesem Ausführungsbeispiel sind die erste Position 311.1 und die zweite Position 311.2 des Daumens 301 jeweils durch die Position des Mittelpunktes der ersten Positionsmarkierung 321 gegeben, die erste Position 312.1 und die zweite Position 312.2 des Zeigefingers 302 sind durch die Position des Mittelpunktes der zweiten Positionsmarkierung 322 gegeben.

Im dargestellten Ausführungsbeispiel handelt es sich bei der ersten Positionsmarke 321 und bei der zweiten Positionsmarke 322 um optische Markierungen durch eine kleine Kugel, die farblich von der Hand 300 der Person 701 abgehoben ist und daher leicht in einem optischen Bild erkannt werden kann. Alternativ kann eine optische Markierung auch durch im sichtbaren Spektrum nicht wahrnehmbare Markierung (z.B. Farbe, die im ultravioletten oder im infraroten Teil des Spektrums reflektiert) realisiert sein, oder durch ein vorgegebenes Farbmuster, oder ebenfalls alternativ Licht ausstrahlen. Weisen die erste Positionsmarke 321 und/oder die zweite Positionsmarke 322 um wenigstens eine Rotationsachse keine Rotationssymmetrie auf, kann die Position und die Orientierung der jeweiligen Positionsmarke schon auf der Basis von weniger optischen Bildern bestimmt werden.

Fig. 6 zeigt einen Werkzeugtisch 600 mit Werkzeugen 601, 602, 603. Bei den Werkzeugen 601, 602, 603 handelt es sich hier um medizinisch-chirurgische Werkzeuge, dargestellt sind hier ein Skalpell 601, eine Schere 602 und eine Pinzette 603. An allen dargestellten Werkzeugen 601, 602, 603 ist eine Werkzeugpositionsmarke 611, 612, 613 angeordnet. Bei der Werkzeugpositionsmarke 611, 612, 613 handelt es sich in diesem Ausführungsbeispiel um einen RFID-Transponder. Die Werkzeugpositionsmarken 611, 612, 613 sind in diesem Ausführungsbeispiel nahe den Bereichen angeordnet, an denen die Werkzeuge 601, 602, 603 typischerweise mit der Hand 300 aufgenommen werden. Die Position bzw. die Koordinaten der Werkzeugpositionsmarken 611, 612, 613 werden in diesem Ausführungsbeispiel mittels des zweiten Positionsdetektors 260 bestimmt, bei dem es sich um ein RFID-Lesegerät handelt.

Alternativ können an einem oder mehreren der Werkzeuge 601, 602, 603 auch mehrere Werkzeugpositionsmarken 611, 612, 613 angeordnet sein. Die Position des Werkzeuges 601, 602, 603 ist in diesem Fall durch die Position eine der am Werkzeug 601, 602, 603 angeordneten Werkzeugpositionsmarken 611, 612, 613 definiert. Die Verwendung von mehreren Werkzeugpositionsmarken 611, 612, 613 ist insbesondere bei großen Werkzeugen 601, 602, 603 und bei multifunktionalen Werkzeugen 601, 602, 603 vorteilhaft. Insbesondere kann bei der Verwendung von mehreren Werkzeugpositionsmarken 611, 612, 613 bereits basierend auf den Koordinaten der Werkzeugpositionsmarken 611, 612, 613 die Orientierung des Werkzeuges 601, 602, 603 bestimmt werden.

Fig. 7 zeigt eine Ansicht einer Anordnung zum Aufzeichnen einer Zuordnung zwischen Greifgesten 1001.1, ..., 1001.N und Werkzeugen 601, 602, 603 von oben. Dargestellt ist die Person 701, deren Bewegungen und Werkzeugaufnahmen aufgezeichnet werden und in ein Training oder eine Schulung überführt werden. Weiterhin ist eine Patientenlagerungsvorrichtung 703 mit einem Patienten 702 abgebildet. Beim Patient 702 kann es sich um eine reale Person handeln, weiterhin kann es sich alternativ um eine Personenattrappe handeln. Weiterhin alternativ kann der Patient 702 auch nur mittels einer Sichtvorrichtung der Person 701 in eine virtuelle Realität, in eine gemischte Realität oder in eine erweiterte Realität für die Person 701 dargestellt werden. Weiterhin ist in der Fig. 6 ein Werkzeugtisch 600 abgebildet, auf dem Werkzeuge 601, ..., 603 abgelegt sind. Bei dem Werkzeugtisch 600 und den Werkzeugen 601, ..., 603 kann es sich wiederum um reale Werkzeuge, um Attrappen oder um Darstellungen mittels einer Sichtvorrichtung der Person 701 in eine virtuelle Realität, in eine gemischte Realität oder in eine erweiterte Realität für die Person 701. Der Positionsdetektor 240 umfasst im dargestellten Ausführungsbeispiel vier einzelne Teildetektoren 240.1, ..., 240.4, mit dieser Vielzahl von Teildetektoren 240.1, ..., 240.4 ist eine gute Erfassung der Positionen der Hand 300 bzw. des Daumens 301 und des Zeigefingers 302 möglich. Alternativ und gleichwertig können noch andere Anzahlen und Anordnungen von Teildetektoren 240.1, ..., 240.4 verwendet werden.

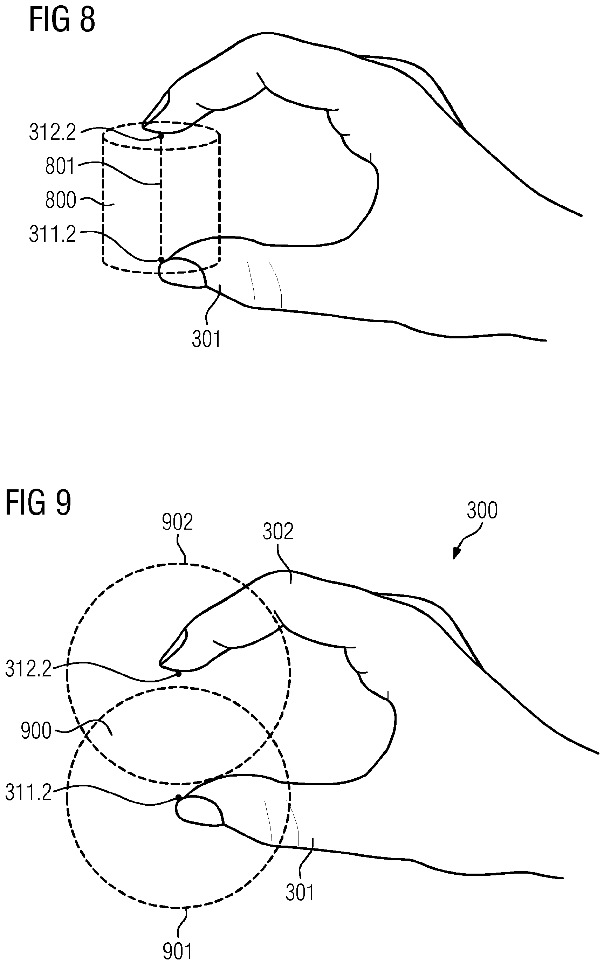

Fig. 8 zeigt ein erstes Ausführungsbeispiel eines Bereichs 800 von Werkzeugpositionen, die einer Greifgeste zugeordnet werden können, Fig. 9 zeigt ein zweites Ausführungsbeispiel eines Bereichs 900 von Werkzeugpositionen, die einer Greifgeste zugeordnet werden können.

Der Bereich 800 des ersten Ausführungsbeispiels ist definiert als ein Zylinder, dessen Vorzugsachse der Strecke 801 zwischen der zweiten Position 311.2 des Daumens 301 und der zweiten Position 312.2 des Zeigefingers 302 entspricht. Ein erstes Werkzeug 601, 602, 603 wird also einer Greifgeste zugeordnet, wenn die Werkzeugposition zum Zeitpunkt der Greifgeste innerhalb des Zylinders angeordnet ist. In anderen Worten wird also ein erstes Werkzeug 601, 602, 603 der Greifgeste zugeordnet, wenn die Werkzeugposition zum Zeitpunkt der Greifgeste zur Strecke 801 zwischen der zweiten Position 311.2 des Daumens 301 und der zweiten Position 312.2 des Zeigefingers 302 einen Abstand aufweist, der kleiner als ein vorgegebener Abstandsschwellenwert ist, und deren Abstand zu der zweiten Position 311.2 des Daumens 301 größer oder gleich dem Abstand zu der Strecke 801 ist, und deren Abstand zu der zweiten Position 312.2 des Zeigefingers 302 größer oder gleich dem Abstand zu der Strecke 801 ist.

Der Bereich 900 des ersten Ausführungsbeispiels ist als Menge aller Punkte definiert, deren Abstand von der zweiten Position 311.2 des Daumens 301 kleiner ist als ein vorgegebener Abstandsschwellenwert, und deren Abstand von der zweiten Position 312.2 des Zeigefingers 302 gleichzeitig kleiner ist als der vorgegebene Abstandsschwellenwert. Der Bereich 900 entspricht in diesem Fall also dem Schnittvolumen einer ersten Kugel 901 und einer zweiten Kugel 902, wobei die erste Kugel 901 eine Kugel um die zweite Position 311.2 des Daumens 301 ist, wobei die zweite Kugel 902 eine Kugel um die zweite Position 312.2 des Zeigefingers 302 ist, und wobei die erste Kugel 901 und die zweite Kugel 902 einen Radius aufweisen, der dem vorgegebenen Abstandsschwellenwert entspricht.

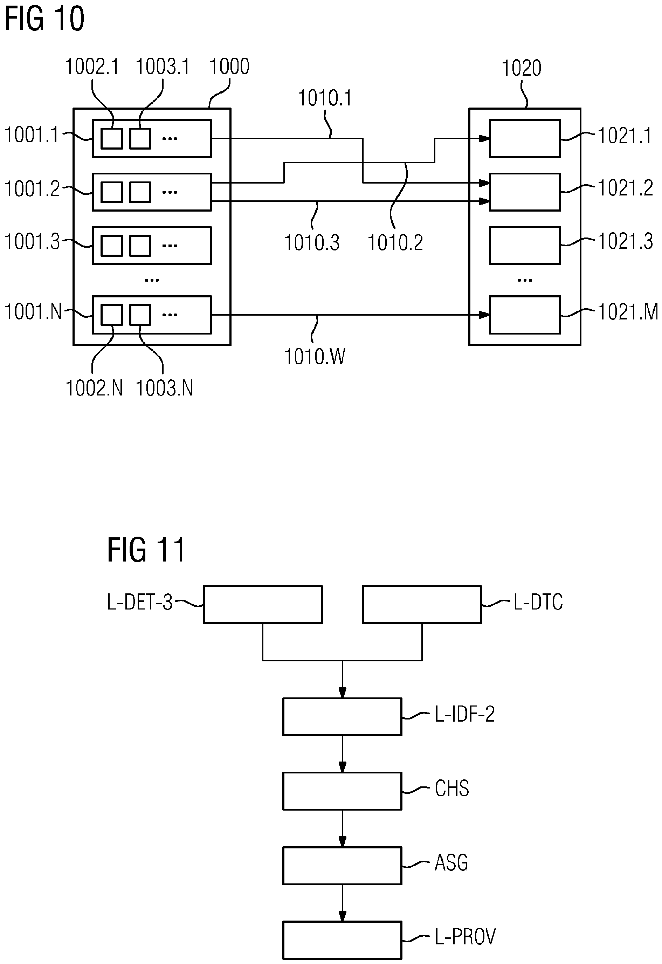

Fig. 10 zeigt eine Zuordnung zwischen Greifgestendatensätzen 1001.1, ..., 1001.N und Werkzeugdatensätzen 1021.1, ..., 1021.M, wobei ein Greifgestendatensatz 1001.1, ..., 1001.N einer Greifgeste zugeordnet werden kann, und ein Werkzeugdatensatz 1021.1, ..., 1021.M einem ersten Werkzeug 601, 602, 603 zugeordnet werden kann. Im dargestellten Ausführungsbeispiel wurden N Greifgestendatensätze 1001.1, ..., 1001.N registriert und in einer Greifgestenliste 1000 gespeichert. Im dargestellten Ausführungsbeispiel sind die M Werkzeugdatensätze 1021.1, ..., 1021.M in einer Werkzeugliste 1020 gespeichert, weiterhin entspricht die Anzahl M der Werkzeugdatensätze 1021.1, ..., 1021.M (und damit auch die Länge M der Werkzeugliste 1020) der Anzahl der Werkzeuge 601, 602, 603, mit denen der Anwender interagieren kann.

Im Allgemeinen kann einer Greifgeste ein Greifgestenzeitpunkt 1002.1, ..., 1002.N und eine Greifgestenposition 1003.1, ..., 1003.N zugeordnet werden. Im dargestellten Ausführungsbeispiel umfasst der Greifgestendatensatz 1001.1, ..., 1001.N den Greifgestenzeitpunkt 1002.1, ..., 1002.N und die Greifgestenposition 1003.1, ..., 1003.N der zugeordneten Greifgeste.

Hierbei kann der Greifgestenzeitpunkt 1002.1, ..., 1002.N auf dem ersten Zeitpunkt oder dem zweiten Zeitpunkt basieren, insbesondere kann der Greifgestenzeitpunkt 1002.1, ..., 1002.N zwischen dem ersten Zeitpunkt und dem zweiten Zeitpunkt liegen, oder insbesondere auch mit dem ersten Zeitpunkt oder dem zweiten Zeitpunkt identisch sein. Im dargestellten Ausführungsbeispiel ist der Greifgestenzeitpunkt 1002.1, ..., 1002.N mit dem zweiten Zeitpunkt identisch.

Die Greifgestenposition 1003.1, ..., 1003.N kann auf der ersten Position 311.1 des Daumens 301 und/oder der ersten Position des 312.1 Zeigefingers 302 basieren. Alternativ und wie im dargestellten Ausführungsbeispiel dargestellt kann die Greifgestenposition 1003.1, ..., 1003.N auch auf der zweiten Position 311.2 des Daumens 301 und der zweiten Position 312.2 des Zeigefingers 302 basieren. Insbesondere kann die Greifgestenposition 1003.1, ..., 1003.N auf der Strecke zwischen der ersten Position 311.1 des Daumens 301 und der ersten Position 312.2 des Zeigefingers 302 oder auf der Strecke zwischen der zweiten Position 311.2 des Daumens 301 und der zweiten Position 312.2 des Zeigefingers 302 angeordnet sein. Insbesondere kann die Greifgestenposition 1003.1, ..., 1003.N auch mit der ersten Position 311.1 oder der zweiten Position 311.2 des Daumens 301 oder mit der ersten Position 312.2 oder der zweiten Position 312.2 des Zeigefingers 302 identisch sein. Alternativ kann die Greifgestenposition 1003.1, ..., 1003.N auch sowohl auf der ersten Position 311.1, 312.1 des Daumens 301 und des Zeigefingers 302 als auch auf der zweiten Position 311.2, 312.2 des Daumens 301 und des Zeigefingers 302 basieren. Insbesondere kann die Greifgestenposition 1003.1, ..., 1003.N als eine gewichtete Summe von Ortsvektoren der Positionen definiert sein, auf denen sie basiert. Im dargestellten Ausführungsbeispiel ist die Greifgestenposition 1003.1, ..., 1003.N der Mittelpunkt von der zweiten Position 311.2 des Daumens 301 und der zweiten Position 312.2 des Zeigefingers 302.

Im dargestellten Ausführungsbeispiel umfasst ein Greifgestendatensatz 1001.1, ..., 1001.N jeweils einen Greifgestenzeitpunkt 1002.1, ..., 1002.N und jeweils eine Greifgestenposition 1003.1, ..., 1003.N. Der Greifgestenzeitpunkt 1002.1, ..., 1002.N entspricht in diesem Beispiel dem zweiten Zeitpunkt der zugeordneten Greifgeste, die Greifgestenposition 1003.1, ..., 1003.N entspricht der zweiten Position 311.2 des Daumens 301 der zugeordneten Greifgeste. Es ist aber alternativ auch möglich, einen anderen Zeitpunkt als Greifgestenzeitpunkt 1002.1, ..., 1002.N oder eine andere Position als Greifgestenposition 1003.1, ..., 1003.N zu verwenden, insbesondere den ersten Zeitpunkt als Greifgestenzeitpunkt 1002.1, ..., 1002.N, oder insbesondere die erste Position 311.1 des Daumens 301, die erste Position 312.1 des Zeigefingers 302 oder die zweite Position 312.2 des Zeigefinger 302 als Greifgestenposition 1003.1, ..., 1003.N.

Im dargestellten Ausführungsbeispiel sind die Greifgestendatensätze 1001.1, ..., 1001.N durch W Zuordnungsrelationen 1010.1, ..., 1010.W zu den Werkzeugdatensätzen 1021.1, ..., 1021.M zugeordnet. Beispielsweise ist der Greifgestendatensatz 1001.1 über die Zuordnungsrelation 1010.1 dem Werkzeugdatensatz 1021.2 zugeordnet, der Greifgestendatensatz 1001.2 ist über die Zuordnungsrelation 1010.2 dem Werkzeugdatensatz 1021.1 und über die Zuordnungsrelation 1010.3 dem Werkzeugdatensatz 1021.2 zugeordnet, und der Greifgestendatensatz 1001.N ist über die Zuordnungsrelation 1010.W dem Werkzeugdatensatz 1021.M zugeordnet.

Es ist möglich, dass ein Greifgestendatensatz 1001.1, ..., 1001.N keinem Werkzeugdatensatz 1021.1, ..., 1021.M zugeordnet ist (z.B. der Greifgestendatensatz 1001.3), in diesem Fall ist der zugehörigen Greifgeste kein erstes Werkzeug 601, 602, 603 zugeordnet. Weiterhin kann ein Greifgestendatensatz 1001.1, ..., 1001.N genau einem Werkzeugdatensatz 1021.1, ..., 1021.M zugeordnet sein (z.B. Greifgestendatensatz 1001.1 oder Greifgestendatensatz 1001.N), in diesem Fall existiert eine eindeutige Zuordnung zwischen der zugehörigen Greifgeste und dem jeweiligen Werkzeug 601, 602, 603. Weiterhin kann ein Greifgestendatensatz 1001.1, ..., 1001.N mehreren Werkzeugdatensätzen 1021.1, ..., 1021.M zugeordnet sein (z.B. Greifgestendatensatz 1001.2), in diesem Fall sind der zugehörigen Greifgeste mehrere Werkzeuge 601, 602, 603 zugeordnet. In besonderen Ausführungsformen darf ein Greifgestendatensatz 1001.1, ..., 1001.N maximal einem Werkzeugdatensatz 1021.1, ..., 1021.M zugeordnet sein, in anderen Ausführungsbeispielen darf ein Greifgestendatensatz 1001.1, ..., 1001.N genau einem Werkzeugdatensatz 1021.1, ..., 1021.M zugeordnet sein.

Die Zuordnungsrelationen 1010.1, ..., 1010.W können insbesondere in Form einer Ablaufliste gespeichert werden. Insbesondere umfasst dann jedes Element dieser Ablaufliste genau einen Greifgestendatensatz 1001.1, ..., 1001.N und eine beliebige Anzahl der Werkzeugdatensätze 1021.1, ..., 1021.M, die dem Greifgestendatensatz 1001.1, ..., 1001.N zugeordnet sind. In bevorzugter Ausführungsform ist die Ablaufliste nach dem Greifgestenzeitpunkt 1002.1, ..., 1002.N der Greifgestendatensätze 1001.1, ..., 1001.N geordnet.

| Tabelle A | |
|---|---|
| A.1 | pos_thb_1 = pos_detector.position_thumb() |
| A.2 | pos_ind_1 = pos_detector.position_index () |
| A.3 | wait (100ms) |
| A.4 | pos_thb_2 = pos_detector.position_thumb() |
| A.5 | pos_ind_2 = pos_detector.position_index() |
| | |
| A.6 | dist_1 = eucl_dist(pos_thb_1, pos_ind_1) |
| A.7 | dist_2 = eucl_dist(pos_thb_2, pos_ind_2) |
| | |
| A.8 | if dist_1 > 0.02 and dist_2 <= 0.02: |
| A.9 |     tool_nrst = tools[0] |
| A.10 |     dist_nrst = eucl_dist(pos_ind_2, tools[0]) |
| A.11 |     for tool in tools: |
| A.12 |         if eucl_dist(pos_ind_2, tool) < dist_nrst: |
| A.13 |             tool_nrst = tool |
| A.14 |             dist_nrst = eucl_dist(pos_ind_2, tool) |
| | |
| A.15 | gestures.store(now(), tool, pos_ind_2) |

Tabelle A zeigt Pseudocode eines Ausführungsbeispiels des erfindungsgemäßen Verfahrens. In den Codezeilen A.1 und A.2 wird mittels von durch den ersten Positionsdetektor 240 "pos_detector" bereitgestellten Funktionen "position_thumb" und "position_index" die erste Position 311.1 des Daumens 301 und die erste Position 312.1 des Zeigefingers 302 zum ersten Zeitpunkt bestimmt. In Codezeile A.3 wird eine Zeitspanne von 100ms gewartet, um in Codezeile A.4 und A.5 die zweite Position 311.2 des Daumens 301 und die zweite Position 312.2 des Zeigefingers 302 zum zweiten Zeitpunkt zu bestimmen. In diesem Ausführungsbeispiel ist der zeitliche Abstand zwischen dem ersten Zeitpunkt und dem zweiten Zeitpunkt daher 100ms. In den Codezeilen A.6 und A.7 wird jeweils der euklidische Abstand zwischen dem Daumen 301 und dem Zeigefinger 302 zum ersten Zeitpunkt und zum zweiten Zeitpunkt bestimmt.

In der Codezeile A.8 wird festgestellt, ob der erste Abstand größer ist als der DZ-Abstandsschwellenwert (hier 2 cm), und ob der zweite Abstand kleiner oder gleich dem DZ-Abstandsschwellenwert ist. Falls dies der Fall ist, wird hier angenommen, dass eine Greifgeste vorliegt.

Aus der Liste der Werkzeugpositionen "tools" wird in Codezeilen A.9 und A.10 zunächst die Werkzeugposition am Anfang der Liste und dessen Abstand zur zweiten Position 312.2 des Zeigefingers 302 bestimmt. Für jede Werkzeugposition der Liste wird dann in Codezeilen A.11 und A.12 bestimmt, ob dessen Abstand kleiner ist als der bisherige kleinste Abstand einer anderen Werkzeugposition. Falls dies der Fall ist, wird diese Werkzeugposition in A.13 und A.14 als neue kleinste Werkzeugposition gesetzt. Die Werkzeugposition kann dabei jeweils immer mit dem zugehörigen Werkzeug 601, 602, 603 und/oder dem zugehörigen Werkzeugdatensatz 1021.1, ..., 1021.M in Verbindung gebracht werden.

In der Codezeile A.15 wird die erkannte Greifgeste (charakterisiert durch den Zeitstempel "now()" der aktuellen Systemzeit) zusammen mit dem Werkzeug 601, 602, 603 bzw. dem Werkzeugdatensatz 1021.1, ..., 1021.M mit dem minimalen Abstand und der zweiten Position 312.2 des Zeigefingers 302 in einer Liste der Greifgesten gespeichert.

| Tabelle B | |
|---|---|
| B.1 | pos_thb_1 = pos_detector.position_thumb() |
| B.2 | pos_ind_1 = pos detector.position index () |
| B.3 | wait(100ms) |
| B.4 | pos_thb_2 = pos_detector.position_thumb () |
| B.5 | pos_ind_2 = pos_detector.position_index () |
| B.6 | dist_1 = eucl_dist(pos_thb_1, pos_ind_1) |
| B.7 | dist_2 = eucl_dist(pos_thb_2, pos_ind_2) |
| B.8 | if dist_1 > 0.02 and dist_2 <= 0.02: |
| B.9 |    tools_assigned = [] |
| B.10 |    for tool in tools: |
| B.11 |       if eucl_dist(pos_ind_2, tool) <= 0.03: |
| B.12 |          tools_assigned.push_back(tool) |
| B.13 |    gestures.store(now(), tools, pos ind 2) |

Tabelle B zeigt Pseudocode eines weiteren Ausführungsbeispiels des erfindungsgemäßen Verfahrens. Die Codezeilen B.1 bis B.9 entsprechen den Codezeilen A.1 bis A.9 der Tabelle A.

Abweichend vom Pseudocode der Tabelle A wird in Codezeile B.9 eine leere Liste "tools assigned" mit Werkzeugen 601 bzw. Werkzeugdatensätzen 1021.1, ..., 1021.M initialisiert, welches eines oder mehrere der Greifgeste zugeordnete Werkzeuge 601, 602, 603 umfasst. Ein Werkzeug 601, 602, 603 wird in Codezeilen B.11 und B.12 dieser Liste zugeordnet, wenn sein euklidischer Abstand von der zweiten Position 312.2 des Zeigefingers 302 kleiner oder gleich 3 cm ist, und wenn sein euklidischer Abstand von der zweiten Position 311.2 des Daumens 301 kleiner oder gleich 3 cm ist. Dies entspricht der in Fig. 9 dargestellten Methode. In der Codezeile B.13 wird die Liste der Werkzeuge 601, 602, 603 bzw. Werkzeugdatensätzen 1021.1, ..., 1021.M analog zu Codezeile A.15 zugeordnet und gespeichert.

Fig. 11 zeigt ein Ausführungsbeispiel eines Verfahrens zum Bereitstellen einer Ablaufliste für eine Anwenderschulung. Der erste Schritt des in Fig. 11 dargestellten Ausführungsbeispiel des Verfahrens ist das Erfassen L-DTC von Bewegungen der Person 701 mittels eines ersten Positionsdetektors 240. In diesem Ausführungsbeispiel umfasst der erste Positionsdetektor 240 ein Motion-Capturing-System, welches mittels einer optischen Erkennung von an der Person 701 angeordneten Positionsmarken 321, 322 die Bewegungen der Person 701 aufzeichnet. Insbesondere werden in diesem Ausführungsbeispiel die Bewegungen des Daumens 301 einer Hand 300 und des Zeigefingers 302 der Hand 300 erfasst. Hierfür kann insbesondere jeweils eine erste Positionsmarke 321 an dem Daumen 301 der Hand 300 und eine zweite Positionsmarke 322 an dem Zeigefinger 302 der Hand 300 angeordnet sein.

Der zweite Schritt des in Fig. 11 dargestellten Ausführungsbeispiel des Verfahrens ist das Dritte Bestimmen L-DET-3 einer Werkzeugmenge, mit denen eine Person 701 bei der Anwenderschulung interagieren kann, mittels eines zweiten Positionsdetektors 260. In dem dargestellten Ausführungsbeispiel umfasst der zweite Positionsdetektor 260 ein RFID-Lesegerät, weiterhin ist an jedem der Werkzeuge 601, 602, 603 eine Werkzeugpositionsmarkierung 611, 612, 613 in Form eines RFID-Transponders angeordnet. Das RFID-Lesegerät kann hierbei die ID von RFID-Transpondern feststellen, deren Abstand vom RFID-Lesegerät kleiner ist als ein Schwellenwert. Anhand der ID der RFID-Transponder kann dann das jeweilige Werkzeug 601, 602, 603 identifiziert werden. Weiterhin ist es mittels dieser Anordnung möglich, auch die Position der RFID-Transponder und damit der Werkzeuge 601, 602, 603 festzustellen.

Der Schritt des Erfassen L-DTC und der Schritt des dritten Bestimmens L-DET-3 sind hierbei unabhängig voneinander und können in einer beliebigen Reihenfolge ausgeführt werden, insbesondere können der Schritt des Erfassen L-DTC und der Schritt des dritten Bestimmens L-DET-3 auch parallel durchgeführt werden.

Der dritte Schritt des in Fig. 11 dargestellten Ausführungsbeispiel des Verfahrens ist das zweite Identifizieren L-IDF-2 einer Greifgeste basierend auf der Bewegung eines Daumens 301 einer Hand 300 der Person 701 und eines Zeigefingers 302 der Hand 300 der Person 701 mittels einer Recheneinheit 222. Im dargestellten Ausführungsbeispiel wird das zweite Identifizieren L-IDF-2 ausgebildet durch die Unterschritte des ersten Bestimmens DET-1, der ersten Berechnens CALC-1, des zweiten Bestimmens DET-2, des zweiten Berechnens CALC-2 und des ersten Identifizierens IDF-1 des im Fig. 1 dargestellten Ausführungsbeispiels eines Verfahrens zum Zuordnen einer Greifgeste zu einem ersten Werkzeug 601, 602, 603.

Der vierte Schritt des in Fig. 11 dargestellten Ausführungsbeispiel des Verfahrens ist das Auswählen CHS eines ersten Werkzeuges 601, 602, 603 aus der Werkzeugmenge basierend auf einer Werkzeugposition des ersten Werkzeuges 601, 602, 603 mittels der Recheneinheit 222, der fünfte Schritt des in Fig. 11 dargestellten Ausführungsbeispiel des Verfahrens ist das Zuordnen ASG des ersten Werkzeuges 601, 602, 603 zu der Greifgeste mittels der Recheneinheit 222. Diese beiden Schritte entsprechen den Schritten des in Fig. 1 dargestellten Ausführungsbeispiels des Verfahrens zum Zuordnen einer Greifgeste zu einem ersten Werkzeug 601, 602, 603.

Der sechste Schritt des in Fig. 11 dargestellten Ausführungsbeispiels des Verfahrens ist das Bereitstellen PROV einer Ablaufliste umfassend wenigstens den Zeitpunkt 1002.1, ..., 1002.N der Greifgeste und das zugeordnete Werkzeug 601, 602, 603 mittels einer Schnittstelle 221. Im dargestellten Ausführungsbeispiel wird die Ablaufliste in Form der in Fig. 10 dargestellten Zuordnungstabelle bereitgestellt, wobei ein Werkzeug 601, 602, 603 durch einen Werkzeugdatensatz 1021.1, ..., 1021.M identifiziert werden kann.

Das in Fig. 11 dargestellte Ausführungsbeispiel des Verfahrens zum Bereitstellen einer Ablaufliste für eine Anwenderschulung kann auf dem in Fig. 2 dargestellten Zuordnungssystem 200 ausgeführt werden, wenn die in Fig. 2 dargestellte Zuordnungssystem 200 dazu ausgebildet ist, die entsprechenden Schritte des Verfahrens zum Bereitstellen einer Ablaufliste für eine Anwenderschulung durchzuführen. Eine solche Zuordnungseinheit kann dann mit einer Bereitstellungseinheit identifiziert werden.

**Patentansprüche**

**1.** Verfahren zum Zuordnen eines ersten Werkzeuges (601, 602, 603) zu einer Greifgeste, umfassend die folgenden Verfahrensschritte:

- Erstes Bestimmen (DET-1) einer ersten Position (311.1) eines Daumens (301) einer Hand (300) einer Person (701) und einer ersten Position (312.1) eines Zeigefingers (302) der Hand (300) zu einem ersten Zeitpunkt mittels eines ersten Positionsdetektors (240),
- Erstes Berechnen (CALC-1) eines ersten Abstandes (315.1) zwischen dem Daumen (301) und dem Zeigefinger (302) zu dem ersten Zeitpunkt basierend auf der ersten Position (311.1) des Daumens (301) und der ersten Position (312.1) des Zeigefingers (302) mittels einer Recheneinheit (222),
- Zweites Bestimmen (DET-2) einer zweiten Position (311.2) des Daumens (301) und einer zweiten Position (312.2) des Zeigefingers (302) zu einem zweiten Zeitpunkt mittels des ersten Positionsdetektors (240), wobei der zweite Zeitpunkt zeitlich nach dem ersten Zeitpunkt liegt,
- Zweites Berechnen (CALC-2) eines zweiten Abstandes (315.2) zwischen dem Daumen (301) und dem Zeigefinger (302) zu dem zweiten Zeitpunkt basierend auf der zweiten Position (311.2) des Daumens (301) und der zweiten Position (312.2) des Zeigefingers (302) mittels der Recheneinheit (222),
- Erstes Identifizieren (IDF-1) einer Greifgeste basierend auf dem ersten Abstand (315.1) und dem zweiten Abstand (315.2) mittels der Recheneinheit (222),
- Auswählen (CHS) eines ersten Werkzeuges (601, 602, 603) aus einer Werkzeugmenge basierend auf einer Werkzeugposition des ersten Werkzeuges (601, 602, 603) mittels der Recheneinheit (222),
- Zuordnen (ASG) des ersten Werkzeuges (601, 602, 603) zu der Greifgeste mittels der Recheneinheit (222).

**2.** Verfahren nach dem Anspruch 1, wobei die Greifgeste identifiziert wird, wenn der erste Abstand (315.1) größer ist als ein DZ-Abstandsschwellenwert und der zweite Abstand (315.2) kleiner oder gleich dem DZ-Abstandsschwellenwert ist.

3. Verfahren nach einem der vorherigen Ansprüche, wobei das Auswählen (CHS) weiterhin auf der ersten Position (311.1) des Daumens (301) und/oder der ersten Position (312.1) des Zeigefingers (302) basiert, oder wobei das Auswählen (CHS) weiterhin auf der zweiten Position (311.2) des Daumens (301) und/oder der zweiten Position (312.2) des Zeigefingers (302) basiert.

4. Verfahren nach Anspruch 3, wobei das erste Werkzeug (601, 602, 603) derart ausgewählt wird, dass der Abstand der Werkzeugposition von der zweiten Position (311.2) des Daumens (301) und/oder von der zweiten Position (312.2) des Zeigefingers (302) kleiner ist als ein FW-Abstandsschwellenwert.

5. Verfahren nach Anspruch 3 oder 4, wobei genau ein erstes Werkzeug (601, 602, 603) ausgewählt wird, und wobei der Abstand des ersten Werkzeuges (601, 602, 603) von der zweiten Position (311.2) des Daumens (301) oder von der zweiten Position (312.2) des Zeigefingers (302) kleiner oder gleich dem Abstand jedes Werkzeuges (601, 602, 603) der Werkzeugmenge von der zweiten Position (311.2) des Daumens (301) oder von der zweiten Position (312.2) des Zeigefingers (302) ist.

6. Verfahren nach einem der vorherigen Ansprüche, wobei die Werkzeugposition basierend auf einer Werkzeugpositionsmarke (611, 612, 613) mittels eines zweiten Positionsdetektors (260) bestimmt wird, wobei die Werkzeugpositionsmarke (611, 612, 613) am ersten Werkzeug (601, 602, 603) angeordnet ist.

7. Verfahren nach einem der Ansprüche 1 bis 5, wobei die Werkzeugposition mittels eines optischen bildgebenden Systems bestimmt wird, wobei die Werkzeugposition auf wenigstens einem optischen Bild des optischen bildgebenden Systems basiert.

8. Verfahren nach einem der vorherigen Ansprüche, wobei das erste Werkzeug (601, 602, 603) aus einer Untermenge der Werkzeugmenge ausgewählt wird, wobei jedes Werkzeug (611, 612, 613) der Untermenge einen Abstand zu der Person (701) aufweist, der kleiner ist als ein PW-Abstandsschwellenwert.

9. Verfahren nach einem der vorherigen Ansprüche, wobei der erste Positionsdetektor (240) ein optisches bildgebendes System umfasst, wobei beim ersten Bestimmen (DET-1) ein erstes optisches Bild aufgenommen wird und die erste Position (311.1) des Daumens (301) und/oder die erste Position (312.1) des Zeigefingers (302) basierend auf dem ersten optischen Bild bestimmt wird, und wobei beim zweiten Bestimmen (DET-2) ein zweites optisches Bild aufgenommen wird und die zweite Position (311.2) des Daumens (301) und/oder die zweite Position (312.2) des Zeigefingers (302) basierend auf dem zweiten optischen Bild bestimmt wird.

10. Verfahren nach einem der vorherigen Ansprüche, wobei an dem Daumen (301) eine erste Positionsmarke (321) angeordnet ist, wobei an dem Zeigefinger (302) eine zweite Positionsmarke (322) angeordnet ist, wobei die erste Position (311.1) und die zweite Position (311.2) des Daumens (301) durch Lokalisierung der ersten Positionsmarke (301) bestimmt werden, und wobei die erste (312.1) und die zweite Position (312.2) des Zeigefingers (302) durch Lokalisierung der zweiten Positionsmarke (322) bestimmt werden.

11. Verfahren nach dem Anspruch 10, wobei der erste Positionsdetektor (240) ein optisches bildgebendes System umfasst, wobei die erste Positionsmarke (321) und die zweite Positionsmarke (322) in einem optischen Bild lokalisiert werden.

12. Zuordnungssystem (200) zum Zuordnen eines ersten Werkzeuges (601, 602, 603) zu einer Greifgeste, umfassend folgende Einheiten:

- Erster Positionsdetektor (240), ausgebildet zum ersten Bestimmen (DET-1) einer ersten Position (311.1) eines Daumens (301) einer Hand (300) einer Person (701) und einer ersten Position (312.1) eines Zeigefingers (302) der Hand (300) zu einem ersten Zeitpunkt, weiterhin ausgebildet zum zweiten Bestimmen (DET-2) einer zweiten Position (311.2) des Daumens (301) und einer zweiten Position (312.2) des Zeigefingers (302) zu einem zweiten Zeitpunkt, wobei der zweite Zeitpunkt zeitlich nach dem ersten Zeitpunkt liegt, - Recheneinheit (222), ausgebildet zum ersten Berechnen (CALC-1) eines ersten Abstandes (315.1) zwischen dem Daumen (301) und dem Zeigefinger (302) zu dem ersten Zeitpunkt basierend auf der ersten Position

(311.1) des Daumens (301) und der ersten Position (312.1) des Zeigefingers (302),

weiterhin ausgebildet zum zweiten Berechnen eines zweiten Abstandes (315.2) zwischen dem Daumen (301) und dem Zeigefinger (302) zu dem zweiten Zeitpunkt basierend auf der zweiten Position (311.2) des Daumens (301) und der zweiten Position (312.2) des Zeigefingers (302), weiterhin ausgebildet zum ersten Identifizieren (IDF-1) einer Greifgeste basierend auf dem ersten Abstand (315.1) und dem zweiten Abstand (315.2) mittels der Recheneinheit (222), weiterhin ausgebildet zum Auswählen (CHS) eines ersten Werkzeuges (601, 602, 603) aus einer Werkzeugmenge basierend auf einer Werkzeugposition des ersten Werkzeuges (601, 602, 603), weiterhin ausgebildet zum Zuordnen (ASG) des ersten Werkzeuges (601, 602, 603) zu der Greifgeste.

13. Zuordnungssystem (200), weiterhin ausgebildet ein Verfahren nach den Ansprüchen 2 bis 11 auszuführen.

14. Computerprogrammprodukt mit einem Computerprogramm, welches direkt in eine Speichereinheit (223) eines Zuordnungssystems (200) ladbar ist, mit Programmabschnitten, um alle Schritte des Verfahrens nach einem der Ansprüche 1 bis 11 auszuführen, wenn die Programmabschnitte von dem Zuordnungssystem (200) ausgeführt werden.

15. Computerlesbares Speichermedium, auf welchem von einem Zuordnungssystem (200) lesbare und ausführbare Programmabschnitte gespeichert sind, um alle Schritte des Verfahrens nach einem der Ansprüche 1 bis 11 auszuführen, wenn die Programmabschnitte von dem Zuordnungssystem (200) ausgeführt werden.

**Geänderte Patentansprüche gemäss Regel 137(2) EPÜ.**

1. Verfahren zum Zuordnen eines ersten Werkzeuges (601, 602, 603) zu einer Greifgeste, umfassend die folgenden Verfahrensschritte:

   - Erstes Bestimmen (DET-1) einer ersten Position (311.1) eines Daumens (301) einer Hand (300) einer Person (701) und einer ersten Position (312.1) eines Zeigefingers (302) der Hand (300) zu einem ersten Zeitpunkt mittels eines ersten Positionsdetektors (240),
   - Erstes Berechnen (CALC-1) eines ersten Abstandes (315.1) zwischen dem Daumen (301) und dem Zeigefinger (302) zu dem ersten Zeitpunkt basierend auf der ersten Position (311.1) des Daumens (301) und der ersten Position (312.1) des Zeigefingers (302) mittels einer Recheneinheit (222),
   - Zweites Bestimmen (DET-2) einer zweiten Position (311.2) des Daumens (301) und einer zweiten Position (312.2) des Zeigefingers (302) zu einem zweiten Zeitpunkt mittels des ersten Positionsdetektors (240),

   wobei der zweite Zeitpunkt zeitlich nach dem ersten Zeitpunkt liegt,

   - Zweites Berechnen (CALC-2) eines zweiten Abstandes (315.2) zwischen dem Daumen (301) und dem Zeigefinger (302) zu dem zweiten Zeitpunkt basierend auf der zweiten Position (311.2) des Daumens (301) und der zweiten Position (312.2) des Zeigefingers (302) mittels der Recheneinheit (222),
   - Erstes Identifizieren (IDF-1) einer Greifgeste basierend auf dem ersten Abstand (315.1) und dem zweiten Abstand (315.2) mittels der Recheneinheit (222),
   - Auswählen (CHS) eines ersten Werkzeuges (601, 602, 603) aus einer Werkzeugmenge basierend auf einer Werkzeugposition des ersten Werkzeuges (601, 602, 603) mittels der Recheneinheit (222),

   wobei das erste Werkzeug (601, 602, 603) ein reales Werkzeug ist,

   - Zuordnen (ASG) des ersten Werkzeuges (601, 602, 603) zu der Greifgeste mittels der Recheneinheit (222).

2. Verfahren nach dem Anspruch 1, wobei die Greifgeste identifiziert wird, wenn der erste Abstand (315.1) größer ist als ein DZ-Abstandsschwellenwert und der zweite Abstand (315.2) kleiner oder gleich dem DZ-Abstandsschwellenwert ist.

3. Verfahren nach einem der vorherigen Ansprüche, wobei das Auswählen (CHS) weiterhin auf der ersten Position (311.1) des Daumens (301) und/oder der ersten Position (312.1) des Zeigefingers (302) basiert, oder wobei das Auswählen (CHS) weiterhin auf der zweiten Position (311.2) des Daumens (301) und/oder der zweiten Position (312.2) des Zeigefingers (302) basiert.

**4.** Verfahren nach Anspruch 3, wobei das erste Werkzeug (601, 602, 603) derart ausgewählt wird, dass der Abstand der Werkzeugposition von der zweiten Position (311.2) des Daumens (301) und/oder von der zweiten Position (312.2) des Zeigefingers (302) kleiner ist als ein FW-Abstandsschwellenwert.

**5.** Verfahren nach Anspruch 3 oder 4, wobei genau ein erstes Werkzeug (601, 602, 603) ausgewählt wird, und wobei der Abstand des ersten Werkzeuges (601, 602, 603) von der zweiten Position (311.2) des Daumens (301) oder von der zweiten Position (312.2) des Zeigefingers (302) kleiner oder gleich dem Abstand jedes Werkzeuges (601, 602, 603) der Werkzeugmenge von der zweiten Position (311.2) des Daumens (301) oder von der zweiten Position (312.2) des Zeigefingers (302) ist.

**6.** Verfahren nach einem der vorherigen Ansprüche, wobei die Werkzeugposition basierend auf einer Werkzeugpositionsmarke (611, 612, 613) mittels eines zweiten Positionsdetektors (260) bestimmt wird, wobei die Werkzeugpositionsmarke (611, 612, 613) am ersten Werkzeug (601, 602, 603) angeordnet ist.

**7.** Verfahren nach einem der Ansprüche 1 bis 5, wobei die Werkzeugposition mittels eines optischen bildgebenden Systems bestimmt wird, wobei die Werkzeugposition auf wenigstens einem optischen Bild des optischen bildgebenden Systems basiert.

**8.** Verfahren nach einem der vorherigen Ansprüche, wobei das erste Werkzeug (601, 602, 603) aus einer Untermenge der Werkzeugmenge ausgewählt wird, wobei jedes Werkzeug (611, 612, 613) der Untermenge einen Abstand zu der Person (701) aufweist, der kleiner ist als ein PW-Abstandsschwellenwert.

**9.** Verfahren nach einem der vorherigen Ansprüche, wobei der erste Positionsdetektor (240) ein optisches bildgebendes System umfasst, wobei beim ersten Bestimmen (DET-1) ein erstes optisches Bild aufgenommen wird und die erste Position (311.1) des Daumens (301) und/oder die erste Position (312.1) des Zeigefingers (302) basierend auf dem ersten optischen Bild bestimmt wird, und wobei beim zweiten Bestimmen (DET-2) ein zweites optisches Bild aufgenommen wird und die zweite Position (311.2) des Daumens (301) und/oder die zweite Position (312.2) des Zeigefingers (302) basierend auf dem zweiten optischen Bild bestimmt wird.

**10.** Verfahren nach einem der vorherigen Ansprüche, wobei an dem Daumen (301) eine erste Positionsmarke (321) angeordnet ist, wobei an dem Zeigefinger (302) eine zweite Positionsmarke (322) angeordnet ist, wobei die erste Position (311.1) und die zweite Position (311.2) des Daumens (301) durch Lokalisierung der ersten Positionsmarke (301) bestimmt werden, und wobei die erste (312.1) und die zweite Position (312.2) des Zeigefingers (302) durch Lokalisierung der zweiten Positionsmarke (322) bestimmt werden.

**11.** Verfahren nach dem Anspruch 10, wobei der erste Positionsdetektor (240) ein optisches bildgebendes System umfasst, wobei die erste Positionsmarke (321) und die zweite Positionsmarke (322) in einem optischen Bild lokalisiert werden.

**12.** Zuordnungssystem (200) zum Zuordnen eines ersten Werkzeuges (601, 602, 603) zu einer Greifgeste, umfassend folgende Einheiten:

- Erster Positionsdetektor (240), ausgebildet zum ersten Bestimmen (DET-1) einer ersten Position (311.1) eines Daumens (301) einer Hand (300) einer Person (701) und einer ersten Position (312.1) eines Zeigefingers (302) der Hand (300) zu einem ersten Zeitpunkt,

weiterhin ausgebildet zum zweiten Bestimmen (DET-2) einer zweiten Position (311.2) des Daumens (301) und einer zweiten Position (312.2) des Zeigefingers (302) zu einem zweiten Zeitpunkt, wobei der zweite Zeitpunkt zeitlich nach dem ersten Zeitpunkt liegt,

- Recheneinheit (222), ausgebildet zum ersten Berechnen (CALC-1) eines ersten Abstandes (315.1) zwischen dem Daumen (301) und dem Zeigefinger (302) zu dem ersten Zeitpunkt basierend auf der ersten Position (311.1) des Daumens (301) und der ersten Position (312.1) des Zeigefingers (302),

weiterhin ausgebildet zum zweiten Berechnen eines zweiten Abstandes (315.2) zwischen dem Daumen (301) und dem Zeigefinger (302) zu dem zweiten Zeitpunkt basierend auf der zweiten Position (311.2) des Daumens (301) und der zweiten Position (312.2) des Zeigefingers (302),

weiterhin ausgebildet zum ersten Identifizieren (IDF-1) einer Greifgeste basierend auf dem ersten Abstand (315.1) und dem zweiten Abstand (315.2) mittels der Recheneinheit (222), weiterhin ausgebildet zum Auswählen (CHS) eines ersten Werkzeuges (601, 602, 603) aus einer Werkzeugmenge basierend auf einer Werkzeugposition des ersten Werkzeuges (601, 602, 603), wobei das erste Werkzeug (601, 602, 603) ein reales Werkzeug ist,

weiterhin ausgebildet zum Zuordnen (ASG) des ersten Werkzeuges (601, 602, 603) zu der Greifgeste.

13. Zuordnungssystem (200) nach dem Anspruch 12, weiterhin ausgebildet ein Verfahren nach den Ansprüchen 2 bis 11 auszuführen.

14. Computerprogrammprodukt mit einem Computerprogramm, welches direkt in eine Speichereinheit (223) eines Zuordnungssystems (200) ladbar ist, mit Programmabschnitten, um alle Schritte des Verfahrens nach einem der Ansprüche 1 bis 11 auszuführen, wenn die Programmabschnitte von dem Zuordnungssystem (200) ausgeführt werden.

15. Computerlesbares Speichermedium, auf welchem von einem Zuordnungssystem (200) lesbare und ausführbare Programmabschnitte gespeichert sind, um alle Schritte des Verfahrens nach einem der Ansprüche 1 bis 11 auszuführen, wenn die Programmabschnitte von dem Zuordnungssystem (200) ausgeführt werden.

FIG 1

```
┌──────────────┐
│              │──── DET-1
└──────────────┘
       │
       ▼
┌──────────────┐
│              │──── CALC-1
└──────────────┘
       │
       ▼
┌──────────────┐
│              │──── DET-2
└──────────────┘
       │
       ▼
┌──────────────┐
│              │──── CALC-2
└──────────────┘
       │
       ▼
┌──────────────┐
│              │──── IDF
└──────────────┘
       │
       ▼
┌──────────────┐
│              │──── CHS
└──────────────┘
       │
       ▼
┌──────────────┐
│              │──── ASG
└──────────────┘
```

FIG 2

```
┌ ─ ─ ─ ─ ─ ─ ─ ─ ─ ─ ─ ─ ─ ─ ─ ─ ─ ─ ┐
  ┌──────────────────┐   ┌──────────┐
│ │ ┌──────────────┐ │   │          │──── 240 │
221 ─│              │ │   │          │
│ │ └──────────────┘ │   └──────────┘      │
  │ ┌──────────────┐ │
222 ─│              │ │                     │
│ │ └──────────────┘ │
  │ ┌──────────────┐ │   ┌──────────┐      │
223 ─│              │ │   │          │──── 260
│ │ └──────────────┘ │   │          │      │
  │ ┌──────────────┐ │   │          │
224 ─│              │ │   └──────────┘      │
│ │ └──────────────┘ │
  └──────────────────┘                     │
└ ─ ─ ─│─ ─ ─ ─ ─ ─ ─│─ ─ ─ ─ ─ ─ ─ ─ ─ ─ ┘
       220           200
```

## FIG 3

300

302

312.1

315.1

311.1

301

## FIG 4

300

302

312.2

315.2

311.2

301

FIG 5

302

300

312.2

322

315.2

321

311.2

301

FIG 6

600

601 611 612 602 613 603

FIG 7

FIG 8

312.2
801
800
311.2

301

FIG 9

902    302    300

312.2

900

311.2

301

901

## FIG 10

## FIG 11

**Europäisches Patentamt**
**European Patent Office**
**Office européen des brevets**

# EUROPÄISCHER RECHERCHENBERICHT

Nummer der Anmeldung

EP 17 18 3006

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 908 215 A1 (SONY CORP [JP]) 19. August 2015 (2015-08-19) | 1-7,9-15 | INV. G06F3/01 |
| A | * Absätze [0016] - [0055]; Abbildungen 1-9 * | 8 | A61B34/00 G06K9/00 |
| | ----- | | G06F3/0481 |
| A | US 2016/210781 A1 (THOMAS MICHAEL [US] ET AL) 21. Juli 2016 (2016-07-21) * Absätze [0001] - [0021] * * Absätze [0072] - [0088]; Abbildung 6 * | 1,12-15 | |
| | ----- | | |
| A | FRANCO TECCHIA ET AL: "I'm in VR!", VIRTUAL REALITY SOFTWARE AND TECHNOLOGY, ACM, 2 PENN PLAZA, SUITE 701 NEW YORK NY 10121-0701 USA, 11. November 2014 (2014-11-11), Seiten 73-76, XP058061755, DOI: 10.1145/2671015.2671123 ISBN: 978-1-4503-3253-8 * Seite 76, linke Spalte * | 2,6,7, 9-11 | |
| | ----- | | **RECHERCHIERTE SACHGEBIETE (IPC)** |
| A | US 2014/157206 A1 (OVSIANNIKOV ILIA [US] ET AL) 5. Juni 2014 (2014-06-05) * Absätze [0032] - [0078]; Abbildungen 1-11 * | 4 | G06F A61B G06K |
| | ----- | | |
| A | US 2006/187196 A1 (UNDERKOFFLER JOHN S [US] ET AL) 24. August 2006 (2006-08-24) * Absätze [0022] - [0040]; Abbildungen 1-2 * * Absatz [0072] * | 6,7,9-11 | |
| | ----- | | |

Der vorliegende Recherchenbericht wurde für alle Patentansprüche erstellt

| Recherchenort | Abschlußdatum der Recherche | Prüfer |
|---|---|---|
| München | 15. Januar 2018 | Legrand, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 17 18 3006

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-01-2018

| Im Recherchenbericht angeführtes Patentdokument | Datum der Veröffentlichung | Mitglied(er) der Patentfamilie | Datum der Veröffentlichung |
|---|---|---|---|
| EP 2908215 A1 | 19-08-2015 | EP 2908215 A1<br>US 2015234467 A1 | 19-08-2015<br>20-08-2015 |
| US 2016210781 A1 | 21-07-2016 | KEINE | |
| US 2014157206 A1 | 05-06-2014 | KR 20140070326 A<br>US 2014157206 A1 | 10-06-2014<br>05-06-2014 |
| US 2006187196 A1 | 24-08-2006 | CN 101536494 A<br>EP 1851750 A2<br>JP 5631535 B2<br>JP 2008530661 A<br>KR 20070116794 A<br>US 2006187196 A1<br>US 2010090946 A1<br>US 2010090947 A1<br>US 2015205364 A1<br>WO 2006086508 A2 | 16-09-2009<br>07-11-2007<br>26-11-2014<br>07-08-2008<br>11-12-2007<br>24-08-2006<br>15-04-2010<br>15-04-2010<br>23-07-2015<br>17-08-2006 |

EPO FORM P0461

Für nähere Einzelheiten zu diesem Anhang : siehe Amtsblatt des Europäischen Patentamts, Nr.12/82